# EUROPEAN PATENT SPECIFICATION

(11) **EP 1 215 208 B1**
(45) Date of publication and mention of the grant of the patent: **12.07.2006**
(21) Application number: 02001881.8
(22) Date of filing: 27.10.1998
(51) Int. Cl.: C07D 277/42, A61K 31/425, C07D 417/12, A61K 31/44, C07D 277/56, C07D 417/06, C07D 417/14, A61K 31/47, A61K 31/50

(54) **4-Aminothiazole derivatives, their preparation and their use as inhibitors of cyclin-dependent kinases**
4-Aminothiazol Derivate, deren Herstellung und deren Verwendung als Inhibitoren Cyclin-abhängiger Kinasen
Dérivées de 4-aminothiazole, leur préparation et leur utilisation comme inhibiteurs des kinases cycline-depenents

(30) Priority: 27.10.1997 US 63634 P; 28.10.1997 US 63666 P
(43) Date of publication of application: 19.06.2002
(62) Divisional of application: 98957393.6
(73) Proprietor: AGOURON PHARMACEUTICALS, INC., La Jolla, CA 92037 (US)
(72) Inventor: Chong, Wesley K.M, Encinitas, California 92024 (US); Chu, Shao Song, Encinitas, California 92024 (US); Duvadie, Rohit R., San Diego, California 92131n (US); Li, Lin, San Diego, California 92131 (US); Xiao, Wei, San Diego, California 92130 (US); Yang, Yi, San Diego, California 92129 (US)
(74) Representative: Hofmann, Harald

(56) References cited:
- WO-A-92/20642
- WO-A-95/15758
- WO-A-96/39145
- GEWALD K. ET AL.: "4-Amino-thiazole" JOURNAL FÜR PRAKTISCHE CHEMIE, vol. 35, no. 4, 1967, pages 97-104, XP002095703
- RAJASEKHARAN K.N. ET AL.: "Studies on the synthesis of 5-acyl-2,4-diaminothiazoles from amidinothioureas" SYNTHESIS, vol. 5, 1986, pages 353-355, XP002095704
- NESTEROV V.N. ET AL.: "Cyclization of nitriles. XXIV. Reactions of cyanamide derivatives of thiocarbamic acids with cyanothioacetamide. Crystal structure of 2-allylamino-4-amino-5-benzoyl-1,3-thiazol e" JOURNAL OF ORGANIC CHEMISTRY, USSR, vol. 24, no. 4, April 1988 (1988-04), pages 762-770, XP002095705
- JENARDANAN G.C. ET AL.: "1-(N-Arylthiocarbamoyl)amidino-3,5-dimeth ylpyrazoles - preparation and use in heterocycle synthesis" SYNTHETIC COMMUNICATIONS, vol. 27, no. 19, 1997, pages 3457-3462, XP002095706
- BINU R. ET AL.: "Synthesis and cyclization of 1-(N-nitroamidino)thioureas to 2,4-diaminothiazoles" ORGANIC PREPARATIONS AND PROCEDURES INTERNATIONAL, vol. 30, no. 1, 1998, pages 93-96, XP002095707

## Description

### Cross-Reference To Related Applications

This regular application claims priority to U.S. Provisional Application No. 60/063,634, filed October 27, 1997, and U.S. Provisional Application No. 60/063,666, filed October 28, 1997.

### Field Of The Invention

This invention is directed to pharmaceutical compositions containing aminothiazole compounds for inhibiting cyclin-dependent kinases (CDKs), such as CDK1, CDK2, CDK4, and CDK6. The invention is also directed to the therapeutic or prophylactic use of pharmaceutical compositions containing such compounds and to methods of treating malignancies and other disorders by administering effective amounts of such compounds.

### Background Of The Invention

Uncontrolled cell proliferation is the insignia of cancer. Cell proliferation in response to various stimuli is manifested by a deregulation of the cell division cycle, the process by which cells multiply and divide. Tumor cells typically have damage to the genes that directly or indirectly regulate progression through the cell division cycle.

CDKs constitute a class of enzymes that play critical roles in regulating the transitions between different phases of the cell cycle, such as the progression from a quiescent stage in G₁ (the gap between mitosis and the onset of DNA replication for a new round of cell division) to S (the period of active DNA synthesis), or the progression from G₂ to M phase, in which active mitosis and cell-division occur. See, e.g., the articles compiled in *Science,* vol. 274 (1996), pp. 1643-1677; and *Ann. Rev. Cell Dev. Biol.,* vol. 13 (1997), pp. 261-291. CDK complexes are formed through association of a regulatory cyclin subunit (e.g., cyclin A, B1, B2, D1, D2, D3, and E) and a catalytic kinase subunit (e.g., cdc2 (CDK1), CDK2, CDK4, CDK5, and CDK6). As the name implies, the CDKs display an absolute dependence on the cyclin subunit in order to phosphorylate their target substrates, and different kinase/cyclin pairs function to regulate progression through specific portions of the cell cycle.

The D cyclins are sensitive to extracellular growth signals and become activated in response to mitogens during the G₁ phase of the cell cycle. CDK4/cyclin D plays an important role in cell cycle progression by phosphorylating, and thereby inactivating, the retinoblastoma protein (Rb). Hypophosphorylated Rb binds to a family of transcriptional regulators, but upon hyperphosphorylation of Rb by CDK4/cyclin D, these transcription factors are released to activate genes whose products are responsible for S phase progression. Rb phosphorylation and inactivation by CDK4/cyclin D permit passage of the cell beyond the restriction point of the G₁ phase, whereupon sensitivity to extracellular growth or inhibitory signals is lost and the cell is committed to cell division. During late G₁, Rb is also phosphorylated and inactivated by CDK2/cyclin E, and recent evidence indicates that CDK2/cyclin E can also regulate progression into S phase through a parallel pathway that is independent of Rb phosphorylation (see Lukas et al., "Cyclin E-induced S Phase Without Activation of the pRb/E2F Pathway," *Genes and Dev.,* vol. 11 (1997), pp. 1479-1492).

The progression from G₁ to S phase, accomplished by the action of CDK4/cyclin D and CDK2/cyclin E, is subject to a variety of growth regulatory mechanisms, both negative and positive. Growth stimuli, such as mitogens, cause increased synthesis of cyclin D1 and thus increased functional CDK4. By contrast, cell growth can be "reined in," in response to DNA damage or negative growth stimuli, by the induction of endogenous inhibitory proteins. These naturally occurring protein inhibitors include p21^{WAF1/CIP1}, p27^{KIP1}, and the p16^{INK4} family, the latter of which inhibit CDK4 exclusively (see Harper, "Cyclin Dependent Kinase Inhibitors," *Cancer Surv.,* vol. 29 (1997), pp. 91-107). Aberrations in this control system, particularly those that affect the function of CDK4 and CDK2, are implicated in the advancement of cells to the highly proliferative state characteristic of malignancies, such as familial melanomas, esophageal carcinomas, and pancreatic cancers (see, e.g., Hall and Peters, "Genetic Alterations of Cyclins, Cyclin-Dependent Kinases, and CDK Inhibitors in Human Cancer," *Adv. Cancer Res.,* vol. 68 (1996), pp.67-108; and Kamb et al., "A Cell Cycle Regulator Potentially Involved in Genesis of Many Tumor Types," *Science,* vol. 264 (1994), pp. 436-440). Over-expression of cyclin D 1 is linked to esophageal, breast, and squamous cell carcinomas (see, e.g., DelSal et al., "Cell Cycle and Cancer: Critical Events at the G₁ Restriction Point," *Critical Rev. Oncogenesis,* vol. 71 (1996), pp. 127-142). Genes encoding the CDK4-specific inhibitors of the p16 family frequently have deletions and mutations in familial melanoma, gliomas, leukemias, sarcomas, and pancreatic, non-small cell lung, and head and neck carcinomas (see Nobori et al., "Deletions of the Cyclin-Dependent Kinase-4 Inhibitor Gene in Multiple Human Cancers," *Nature,* vol. 368 (1994), pp. 753-756). Amplification and/or overexpression of cyclin E has also been observed in a wide variety of solid tumors, and elevated cyclin E levels have been correlated with poor prognosis. In addition, the cellular levels of the CDK inhibitor p27, which acts as both a substrate and inhibitor of CDK2/cyclin E, are abnormally low in breast, colon, and prostate cancers, and the expression levels of p27 are inversely correlated with the stage of disease (see Loda et al., "Increased Proteasome-dependent Degradation of the Cyclin-Dependent Kinase Inhibitor p27 in Aggressive Colorectal Carcinomas," *Nature Medicine,* vol. 3 (1997), pp. 231-234). The p21 proteins also appear to transmit the p53 tumor-suppression signal to the CDKs; thus, the mutation of p53 in approximately 50% of all human cancers may indirectly result in deregulation of CDK activity.

The emerging data provide strong validation for the use of compounds inhibiting CDKs, and CDK4 and CDK2 in particular, as anti-proliferative therapeutic agents. Certain biomolecules have been proposed for this purpose. For example, U.S. Patent No. 5,621,082 to Xiong et al. discloses nucleic acid encoding an inhibitor of CDK6, and European Patent Publication No. 0 666 270 A2 describes peptides and peptide mimetics that act as inhibitors of CDK1 and CDK2. Several small molecules have been identified as CDK inhibitors (for a recent review, see Webster, "The Therapeutic Potential of Targeting the Cell Cycle," *Exp. Opin. Invest. Drugs,* vol. 7 (1998), pp. 865-887). The flavone flavopiridol displays modest selectivity for inhibition of CDKs over other kinases, but inhibits CDK4, CDK2, and CDK1 equipotently, with IC₅₀s in the 0.1-0.3 µM range. Flavopiridol is currently in Phase II clinical trials as an oncology chemotherapeutic (Sedlacek et al., "Flavopiridol (L86-8275; NSC 649890), A New Kinase Inhibitor for Tumor Therapy," *Int. J. Oncol.,* vol. 9 (1996), pp. 1143-1168). Analogs of flavopiridol are the subject of other publications, for example, U.S. Patent No. 5,733,920 to Mansuri et al. (International Publication No. WO 97/16447) and International Publication Nos. WO 97/42949, and WO 98/17662. Results with purine-based derivatives are described in Schow et al., *Bioorg. Med. Chem. Lett*., vol. 7 (1997), pp. 2697-2702; Grant et al., *Proc. Amer. Assoc. Cancer Res*,. vol. 39 (1998), Abst. 1207; Legravend et al., *Bioorg. Med Chem. Lett.,* vol. 8 (1998), pp. 793-798; Gray et al., *Science,* vol. 281 (1998), pp. 533-538; and Furet et al., *216th ACS Natl. Mtg.* (Aug 23-27, 1998, Boston), Abst MEDI-218. In addition, the following publications disclose certain pyrimidines that inhibit cyclin-dependent kinases and growth-factor mediated kinases: International Publication No. WO 98/33798; Ruetz et al., *Proc. Amer. Assoc. Cancer Res,* vol. 39 (1998), Abst. 3796; and Meyer et al., *Proc. Amer. Assoc. Cancer Res.,* vol. 39 (1998), Abst. 3794.

There is still a need, however, for small-molecule compounds that may be readily synthesized and are potent inhibitors of one or more CDKs or CDK/cyclin complexes. Because CDK4 may serve as a general activator of cell division in most cells, and because complexes of CDK4/cyclin D and CDK2/cyclin E govern the early G₁ phase of the cell cycle, there is a need for effective and specific inhibitors of CDK4 and/or CDK2 for treating one or more types of tumors.

### Summary Of The Invention

Accordingly, one object of the invention is to attain compounds and drug compositions that inhibit the activity of one or more CDKs, such as CDK2, CDK4, and/or CDK6, or cyclin complexes thereof. A further object is to provide an effective method of treating cancer indications through CDK inhibition, preferably through inhibition of CDK4 or CDK4/D-type cyclin complexes and/or CDK2 or CDK2/E-type cyclin complexes. Another object is to achieve pharmaceutical compositions containing compounds effective to block the transition of cancer cells into their proliferative phase. These and other objects and advantages of the invention, which will become apparent in light of the detailed description below, are achieved through cell-cycle control agents of the invention described below.

In one general aspect, the invention relates to pharmaceutical compositions comprising:
(a) a cell-cycle control agent selected from:
   (i) compounds of the Formula I: 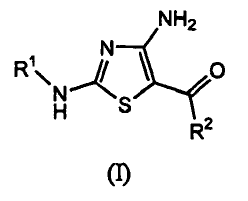 wherein:
      R¹ is selected from: 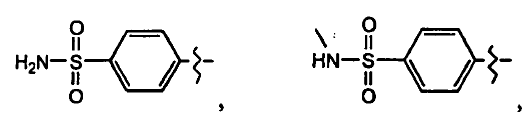 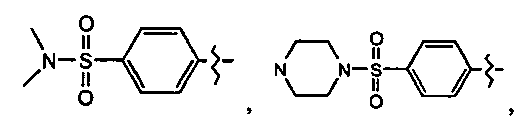 and 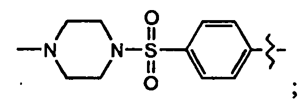 and
      R² is a substituted or unsubstituted: carbocyclic or heterocyclic, monocyclic or fused or non-fused polycyclic, ring structure; where each optional substituent for R² is independently a halogen (e.g., chloro, iodo, bromo, or fluoro); oxygen (=O); haloalkyl (e.g., trifluoromethyl); C₁₋₆-alkyl; C₁₋₆-alkenyl; C₁₋₆₋alkynyl; hydroxyl; C₁₋₆-alkoxyl; carbocyclic cycloallcyl, which may be monocyclic or fused or non-fused polycyclic (e.g., cyclopropyl, cyclobutyl, cyclopentyl, or cyclohexyl), or a heterocycloalkyl, which may be monocyclic or fused or non-fused polycyclic (e.g., pyrrolidinyl, piperidinyl, piperazinyl, morpholinyl, or thiazinyl); carbocyclic or heterocyclic, monocyclic or fused or non-fused polycyclic aryl (e.g., phenyl, naphthyl, pyrrolyl, indolyl, furanyl, thiophenyl, imidazolyl, oxazolyl, isoxazolyl, thiazolyl, triazolyl, tetrazolyl, pyrazolyl, pyridinyl, quinolinyl, isoquinolinyl, acridinyl, pyrazinyl, pyridazinyl, pyrimidinyl, benzimidazolyl, benzothiophenyl, or benzofuranyl); amino (primary, secondary, or tertiary); nitro; thiol; thioether; imine; cyano; amido; phosphonato; phosphine; carboxyl; thiocarbonyl; sulfonyl; sulfonamide; ketone; aldehyde; or ester;
   (ii) pharmaceutically acceptable salts of compounds of the Formula I; and
   (iii) prodrugs and pharmaceutically active metabolites of compounds of the Formula I or pharmaceutically acceptable salts thereof; and
(b) a pharmaceutically acceptable carrier.

Such compositions are useful as inhibitors of mammalian CDK/cyclin complexes, insect CDK, or fungal CDK complexes. Such compositions are also useful for controlling proliferation, differentiation, and/or apoptosis. Thus, in one general aspect the invention is directed to pharmaceutical compositions containing pharmaceutically effective amounts of cell-cycle control agents.

In a preferred embodiment, the invention is directed to potent cell-cycle control agents where R² in Formula I is an ortho-substituted aryl ring structure (e.g., *o*-substituted phenyl). Particularly preferred among such agents are those in which R² is an *o-*disubstituted phenyl.

The invention further relates to methods of using cell-cycle control agents for treating diseases or disorders mediated by CDK inhibition, especially those mediated by CDK4 and/or CDK2 inhibition. More particularly, the invention is directed to methods of treating malignancies or cancer-type disorders by administering a pharmaceutical composition comprising a cell-cycle control agent. Additionally, the invention relates to the use of cell-cycle control agents to prevent or treat mycotic infections.

Other aspects, advantages, and preferred features of the invention will become apparent from the detailed description below.

### Detailed Description And Preferred Embodiments Of The Invention

In one general embodiment, the invention relates to pharmaceutical compositions each comprising:
(a) an amount of a cell-cycle control agent effective to inhibit a CDK, the cell-cycle control agent being:
   (i) a compound of the Formula I: 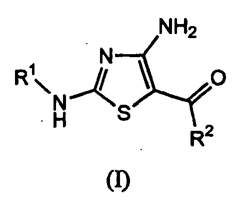 wherein:
      R¹ is a substituted or unsubstituted group selected from: sulfonyl; and
      R² is a substituted or unsubstituted, carbocyclic or heterocyclic, monocyclic or fused or non-fused polycyclic, ring structure;
      where each optional substituent for R¹ and R² is independently a halogen; haloalkyl; C₁₋₆-alkyl; C₁₋₆-alkenyl; C₁₋₆-alkynyl; hydroxyl; C₁₋₆ alkoxyl; amino; nitro; thiol; thioether; imine; cyano; amido; phosphonato; phosphine; carboxyl; thiocarbonyl; sulfonyl; sulfonamide; ketone; aldehyde; or ester;
   (ii) a pharmaceutically acceptable salt of a compound of the Formula I; or
   (iii) a prodrug or pharmaceutically active metabolite of a compound of the Formula I or a pharmaceutically acceptable salt thereof; and
(b) a pharmaceutically acceptable carrier.

In another general embodiment, each optional substituent for R¹ and R² may be independently selected from, in addition to the above-identified groups, the following groups: oxygen; carbocyclic or heterocyclic, monocyclic or fused or non-fused polycyclic, cycloalkyl; and carbocyclic or heterocyclic, monocyclic or fused or non-fused polycyclic, aryl. Such substituents may optionally be further substituted with a substituent selected from such groups.

Other particularly preferred R¹ groups include phenyl groups substituted with carbonyl or sulfonamide moieties, wherein the carbonyl carbon and sulfonamide nitrogen are optionally further substituted. The following are examples of preferred R¹ groups: 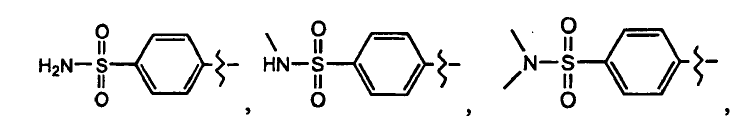 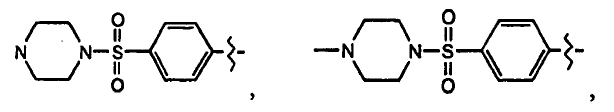 , where R³ is selected from C₁-C₆ alkyl, C₁-C₆ alkoxy, aryl, aryloxy, and amine.

In preferred embodiments, R² in Formula I is a bulky group such as a substituted or unsubstituted carbocyclic or heterocyclic monocycle, or a substituted or unsubstituted fused or non-fused carbocyclic or heterocyclic polycycle. More preferably, R² is a substituted (carbo or poly)-(monocycle or polycycle); even more preferably, R² is such a cyclic ring structure bearing a substituent at the position adjacent or vicinal to the point of attachment (to the core structure).

For example, preferred species for R² include an ortho-substituted aromatic ring structure such as o-substituted phenyl or thienyl, or a 1,2-substituted cycloalkyl or cycloalkenyl ring structure such as 2-substituted cyclopent-1-enyl. Particularly preferred examples for the moiety R² include substituted or unsubstituted: *o*-halophenyl (e.g., *o-*fluorophenyl, *o*-chlorophenyl, *o*-iodophenyl, or *o*-bromophenyl), *o*-nitrophenyl, *o-*aminophenyl, *o*-C₁₋₆-alkylphenyl, *o*-C₁₋₆-alkoxyphenyl (e.g., *o*-methoxyphenyl or *o-*ethoxyphenyl), *o*-C₁₋₆-alkoxybenzothiophenyl, *o*-methylthiophenyl, benzonitrile, and carboxybenzyl. Particularly preferred examples for the moiety R² also include ortho-disubstituted aryls, for example, 2,6-dihalophenyl (e.g., 2,6-difluorophenyl) and 2-halo-6-trifluoromethylphenyl (e.g., 2-fluoro-6-trifluommethylphenyl). Compounds of the Formula I where R² is a 1,2-substituted cyclic ring structure, optionally having one or more additional substituents, such as an ortho-substituted aryl having another substituent at the para position, have been surprisingly found to be potent CDK inhibitors.

Particularly preferred examples of compounds of Formula I include:

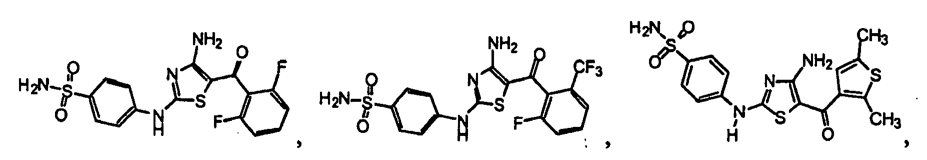

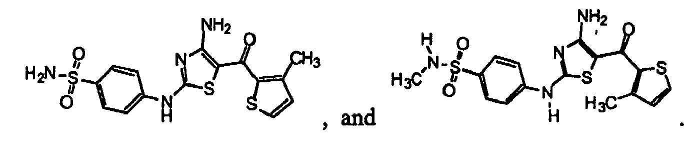

Pharmaceutical compositions according to the invention may, alternatively or in addition to a compound of the Formula I, comprise as an active ingredient a pharmaceutically acceptable salt of a compound of the Formula I, or a prodrug or pharmaceutically active metabolite of such a compound or salt. Such compounds, salts, prodrugs, and metabolites are sometimes referred to herein collectively as "cell-cycle control agents."

Compositions in accordance with the invention inhibit the kinase activity of CDK/cyclin complexes, such as those active in the G₀ or G₁ stage of the cell cycle, e.g., CDK2, CDK4, and/or CDK6 complexes. Preferred compositions of the invention contain cell-cycle control agents having an inhibition constant against CDK4 or a CDK4/D-type cyclin complex of about 1 µM or less, more preferably of about 500 nM or less, even more preferably of about 200 nM or less, and most preferably of about 100 nM or less. Especially preferred compounds of the invention include those having a CDK4/cyclin D3 inhibition constant (Kᵢ CDK4/D3) of about 100 nM or less. Other preferred compositions of the invention contain cell-cycle control agents having an inhibition constant against CDK2 or a CDK2/E-type cyclin complex of about 1 µM or less, more preferably of about 500 nM or less, even more preferably of about 200 nM or less, and most preferably of about 100 nM or less.

Certain compounds of the Formula I may exist in various stereoisomeric or tautomeric forms. The present invention encompasses all such CDK-inhibiting compounds, including active compounds in the form of essentially pure enantiomers, racemic mixtures, and tautomers.

The term "pharmaceutically acceptable" means pharmacologically acceptable and substantially non-toxic to the subject being administered the cell-cycle control agent. Pharmaceutically acceptable salts include conventional acid-addition salts or base-addition salts formed from suitable non-toxic organic or inorganic acids or inorganic bases. Exemplary acid-addition salts include those derived from inorganic acids such as hydrochloric acid, hydrobromic acid, hydroiodic acid, sulfuric acid, sulfamic acid, phosphoric acid, and nitric acid, and those derived from organic acids such as p-toluenesulfonic acid, methanesulfonic acid, ethane-disulfonic acid, isethionic acid, oxalic acid, p-bromophenylsulfonic acid, carbonic acid, succinic acid, citric acid, benzoic acid, 2-acetoxybenzoic acid, acetic acid, phenylacetic acid, propionic acid, glycolic acid, stearic acid, lactic acid, malic acid, tartaric acid, ascorbic acid, maleic acid, hydroxymaleic acid, glutamic acid, salicylic acid, sulfanilic acid, and fumaric acid. Exemplary base-addition salts include those derived from ammonium hydroxides (e.g., a quaternary ammonium hydroxide such as tetramethylammonium hydroxide), those derived from inorganic bases such as alkali or alkaline earth-metal (e.g., sodium, potassium, lithium, calcium, or magnesium) hydroxides, and those derived from organic bases such as carbonates, bicarbonates, amines, benzylamines, piperidines, and pyrrolidines.

The term "prodrug" refers to a metabolic precursor of a compound of the Formula I (or a salt thereof) that is pharmaceutically acceptable. A prodrug may be inactive when administered to a subject but is converted *in vivo* to an active compound of the Formula I. The term "active metabolite" refers to a metabolic product of a compound of the Formula I that is pharmaceutically acceptable and effective. Prodrugs and active metabolites of compounds of the Formula I may be determined using techniques known in the art.

Cell-cycle control agents in accordance with the invention are useful as pharmaceuticals for treating proliferative disorders in mammals, especially humans, marked by unwanted proliferation of endogenous tissue. Compounds of the Formula I may be used for treating subjects having a disorder associated with excessive cell proliferation, e.g., cancers, psoriasis, immunological disorders involving undesired proliferation of leukocytes, and restenosis and other smooth-muscle disorders. Furthermore, such compounds may be used to prevent de-differentiation of post-mitotic tissue and/or cells.

Pharmaceutical compositions or preparations of the invention comprise a pharmaceutically acceptable carrier and an effective amount of at least one cell-cycle control agent. The term "effective amount" means an amount that significantly inhibits proliferation and/or prevents de-differentiation of a eukaryotic cell, e.g., a mammalian, insect, plant, or fungal cell, and is effective for the indicated utility, e.g., specific therapeutic treatment.

The specific dosage amount of a cell-cycle control agent being administered to obtain therapeutic or inhibitory effects, of course, may be determined in a manner known in the art according to the particular circumstances surrounding the case, including, e.g., the specific agent being administered, the route of administration, the condition being treated, and the subject or host being treated. An exemplary total daily dose of a cell-cycle control agent, which may be administered in single or multiple doses, contains a dosage level of from about 0.01 mg/kg body weight to about 50 mg/kg body weight.

The cell-cycle control agents of the invention may be administered by any of a variety of suitable routes, such as orally, rectally, transdermally, subcutaneously, intravenously, intramuscularly, or intranasally. The cell-cycle control agents are preferably formulated into compositions suitable for the desired routes before being administered.

A pharmaceutical composition or preparation according to the invention comprises an effective amount of a cell-cycle control agent and a pharmaceutically acceptable carrier, such as a diluent or excipient for the agent. When the carrier serves as a diluent, it may be a solid, semi-solid, or liquid material acting as a vehicle, excipient, or medium for the active ingredient(s). Compositions according to the invention may be made by admixing the active ingredient(s) with a carrier, or diluting it with a carrier, or enclosing or encapsulating it within a carrier, which may be in the form of a capsule, sachet, paper container, or the like. Exemplary ingredients, in addition to one or more cell-cycle control agents and any other active ingredients, include Avicel (microcrystalline cellulose), starch, lactose, calcium sulfate dihydrate, terra alba, sucrose, talc, gelatin, agar, pectin, acacia, magnesium stearate, stearic acid, peanut oil, olive oil, glyceryl monostearate, Tween 80 (polysorbate 80), 1,3-butanediol, cocoa butter, beeswax, polyethylene glycol, propylene glycol, sorbitan monostearate, polysorbate 60, 2-octyldodecanol, benzyl alcohol, glycine, sorbic acid, potassium sorbate, disodium hydrogen phosphate, sodium chloride, and water.

The compositions may be prepared in any of a variety of forms suitable for the desired mode of administration. For example, pharmaceutical compositions may be prepared in the form of tablets, pills, powders, lozenges, sachets, cachets, elixirs, suspensions, emulsions, solutions, syrups, aerosols (as solids or in liquid media), ointments (e.g., containing up to 10% by weight of a cell-cycle control agent), soft-gel and hard-gel capsules, suppositories, sterile injectable solutions, sterile packaged powders, and the like.

A pharmaceutical composition according to the invention comprises a cell-cycle control agent and, optionally, one or more other active ingredients, such as a known antiproliferative agent that is compatible with the cell-cycle control agent and suitable for the indication being treated. In a preferred embodiment, a pharmaceutical composition of the invention includes an effective amount of a cell-cycle control agent of the Formula I as an active ingredient.

Compounds in accordance with the invention may be prepared in manners analogous to those specifically described below, with the lettered example prefixes (i.e., A, B, C, D, E, F, G, H, J, K, L, M and N) designating general synthesis schemes.

### EXAMPLES

- Example C(33): 4-[4-Amino-5-(2,4-dimethoxy-benzoyl)-thiazol-2-ylamino]-benzenesulfonamide 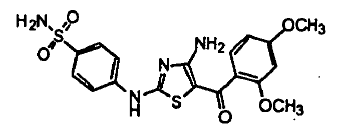
   The title compound was prepared essentially as described for Example C(1). 4-Isothiocyanato-benzenesulfonamide and 2-bromo-2',4'-dimethoxyacetophenone provided 75% yield of yellow powder, mp 249-250°C.
   ¹H NMR (DMSO-d₆): δ 10.93 (1H, bs), 7.93 (2H, bs), 7.75 (4H, bs), 7.25 (2H, bs), 7.21 (1H, d, J = 8.1 Hz), 6.61 (1H, d, J = 1.9 Hz), 6.55 (1H, dd, J = 8.1, 1.9 Hz), 3.79 (3H, s), 3.76 (3H, s).
   FABMS (MH⁺): 435.
   Anal. Calcd. for C₁₈H₁₈N₄O₅S₂: C, 49.76; H, 4.18; N, 12.89; S, 14.76. Found: C, 49.66; H, 4.15; N, 12.77; S, 14.86.
- Example C(34): Ethyl 4-[4-Amino-2-(4-sulfamoyl-phenylamino)-thiazole-5-carbonyl]-benzoate 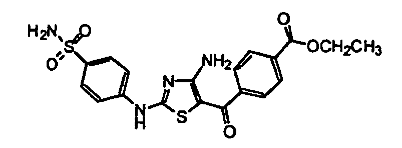
   The title compound was prepared substantially as described for Example C(1). 4-Isothiocyanato-benzenesulfonamide and ethyl 4-bromoacetyl-benzoate provided 95% yield of yellow powder, mp 225-227°C.
   ¹H NMR (DMSO-d₆): δ 11.16 (1H, s), 8.32 (2H, bs), 8.04 (2H, d, J = 8.4 Hz), 7.80 (2H, d, J = 8.4 Hz), 7.78 (4H, bs), 7.26 (2H, bs), 4.33 (2H, q, J = 7.2 Hz), 1.33 (3H, t, J = 7.2 Hz).
   FABMS (MH⁺): 447.
   Anal. Calcd. for C₁₉H₁₈N₄O₅S₂ • 0.4 H₂O: C, 50.30; H, 4.18; N, 12.38; S, 14.13.
   Found: C, 50.11; H, 3.97; N, 12.26; S, 14.14.
- Example C(35): 4-[4-Amino-5-(2,4-dimethyl-benzoyl)-thiazol-2-ylamino]-benzenesulfonamide 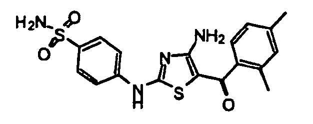
   The title compound was prepared essentially as described for Example C(1). 4-Isothiocyanatobenzenesulfonamide and 2-bromo-2',4'-dimethylacetophenone furnished a yellow solid in 75% yield, mp 242-244°C.
   ¹H NMR (DMSO-d₆): δ 10.97 (1H, bs), 8.00 (2H, bs), 7.76 (2H, d, J = 9.7 Hz), 7.72 (2H, d, J = 9.7 Hz), 7.24 (2H, bs), 7.22 (1H, d, J = 7.5 Hz), 7.07 (1H, s), 7.03 (1H, d, J = 7.5 Hz), 2.29 (3H, s), 2.23 (3H, s).
   FABMS (MH⁺): 403.
   Anal. Calcd. for C₁₈H₁₈N₄O₃S₂: C, 53.71; H, 4.51; N, 13.92; S, 15.93. Found: C, 53.47; H, 4.54; N, 13.69; S, 15.83.
- Example C(38): 4-[4-Amino-5-(2,6-dichloro-4-trifluommethyl-benzoyl)-thiazol-2-ylamino]-benzenesulfonamide 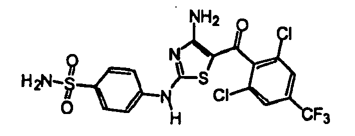
   The title compound was prepared essentially as described for Example C(1). 4-Isothiocyanatobenzenesulfonamide and 2-bromo-2',6'-dichloro-4'-(trifluoromethyl) acetophenone furnished, after recrystallization from EtOH/H₂O and drying via benzene azeotrope, a yellow solid in 46% yield, mp 294-296°C.
   ¹H NMR (DMSO-d₆): δ 8.10 (1H, s), 8.05 (2H, s) 7.77 (4H, dd, J = 9.0, 14.0 Hz).
   HRFABMS: Calcd. for C₇H₁₂Cl₂F₃N₄O₃S₂ (MH⁺): 510.9680. Found: 510.9697. Anal. Calcd. for C₁₇H₁₁Cl₂F₃N₄O₃S₂·0.1 H₂O • C₆H₆: C, 40.28; H, 2.51; N, 10.30; S, 11.97; Cl, 13.51. Found: C, 40.58; H, 2.28; N, 10.75; S, 12.31; Cl, 13.61.
- Example C(39): Phenyl 4-[4-Amino-2-(4-sulfamoyl-phenylamino)-thiazole-5-carbonyl]-benzoate 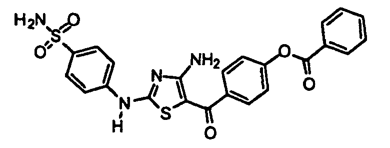
   The title compound was prepared in a manner analogous to that used in Example C(1). 4-Isothiocyanato-benzenesulfonamide and 4-(bromoacetyl)-phenyl benzoate provided a yellow solid in 77% yield, mp >300°C.
   ¹H NMR (DMSO-d₆): δ 11.13 (1H, s), 8.26 (2H, bs), 8.15 (2H, dd, J = 7.2, 1.6 Hz), 7.83-7.73 (7H, m), 7.66-7.59 (2H, m), 7.41 (2H, d, J = 6.9 Hz), 7.27 (2H, s).
   HRFABMS (MH⁺): Calcd.: 495.0797. Found: 495.0812.
   Anal. Calcd. for C₂₃H₁₈N₄O₅S₂ • 0.2 H₂O: C, 55.45; H, 3.72; N, 11.25; S, 12.87. Found: C, 55.34; H, 3.592; N, 11.01; S, 12.88.
- Example C(47): 4-[4-Amino-5-(4-methyl-thiazole-5-carbonyl)-thiazol-2-ylamino]-benzenesulfonamide 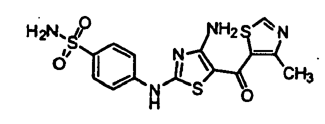
   5-Bromoacetyl-4-methyl-thiazole, which has the structural formula 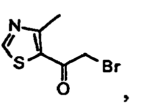 was prepared as described in Sych et al., *J. Gen. Chem. USSR,* vol. 32 (1962), pp. 970-975. Bromine (0.75 mL, 7.77 mmol) was added dropwise into the solution of 1-(4-methyl-thiazol-5-yl)-ethanone (2.05 mg, 14.5 mmol; Ganapathi et al., *Proc.-Indian Acad Sci. Sect. A,* vol. 22 (1945), pp. 362-378) in HOAc (3 mL). The mixture was stirred at 85°C for 1.5 hours and turned into yellow cake. HOAc (3 mL) was added, and after 1.5 hours, allowed to cool. The HOAc was removed *in vacuo* and the residue partitioned between CH₂Cl₂ and sat aq NaHCO₃. The organic layer was washed with brine, dried over Na₂SO₄, and evaporated to give a black solid, 1.3 g (41% yield), which was used without further purification.
   ¹H NMR (CDCl₃): δ 8.85 (1H, s), 4.28 (2H, s), 2.81 (3H, s).
   The title compound was prepared in a manner analogous to that used in Example C(1). 4-Isothiocyanato-benzenesulfonamide and 5-bromoacetyl-4-methyl-thiazole provided a brown solid in 31 % yield, mp 265-266°C.
   ¹H NMR (DMSO-d₆): δ 11.18 (1H, s), 9.08 (1H, s), 8.30 (2H, bs), 7.78 (4H, bs), 7.72 (2H, bs), 2.55 (3H, s).
   Anal. Calcd. for C₁₆H₁₃N₅O₃S₃: C, 42.52; H, 3.31; N, 17.11; S, 24.32. Found: C, 42.28; H, 3.33; N, 17.15; S, 24.52.
- Example C(48): 4-[4-Amino-5-(3-methyl-thiophene-2-carbonyl)-thiazol-2-ylamino]-benzenesulfonamide 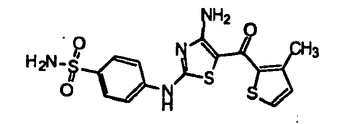
   The title compound was prepared in a manner analogous to that used in Example C(1). 4-Isothiocyanato-benzenesulfonamide and 2-bromoacetyl-3-methyl-thiophene (from Example C(19)) provided a yellow solid in 69% yield, mp 284.5-286.0°C.
   ¹H NMR (DMSO-d_{b}): δ 11.11 (1H, s), 8.20 (2H, bs), 7.80 (2H, d, J =10.7 Hz), 7.76 (2H, d, J =10.7 Hz), 7.61 (1H, d, J = 5.0 Hz), 7.26 (2H, s), 6.90 (1H, d, J = 5.0 Hz), 2.38 (3H, s).
   Anal. Calcd. for C₁₅H₁₄N₄O₃S₃: C, 45.67; H, 3.58; N, 14.20; S, 24.39. Found: C, 45.52; H, 3.58; N, 14.04; S, 24.36.
- Example C(49): 4-[4-Amino-5-(3-methyl-benzo[b]thiophene-2-carbonyl)-thiazol-2-ylamino]-benzenesulfonamide 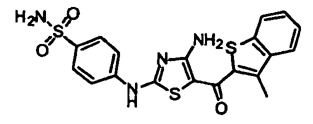
   The title compound was prepared in a manner analogous to that used in Example C(1). 4-Isothiocyanato-benzenesulfonamide and 2-(2-bromoacetyl)-3-methylbenzo[*b*]thiophene provided a yellow powder in 73% yield, mp 274.0-275.5°C.
   ¹H NMR (DMSO-d₆): δ 11.17 (1H, bs), 8.33 (2H, bs), 8.04-7.97 (1H, m), 7.90-7.84 (1H, m), 7.78 (4H, bs), 7.51-7.44 (2H, m), 7.27 (2H, s), 2.52 (3H, s).
   Anal. Calcd. for C₁₉H₁₆N₄O₃S₃: C, 51.33; H, 3.63; N, 12.60; S, 21.64. Found: C, 51.19; H, 3.67; N, 12.31; S, 21.37.
- Example C(50): 4-[4-Amino-5-(2,5-dimethyl-thiophene-3-carbonyl)-thiazol-2-ylamino]-benzenesulfonamide 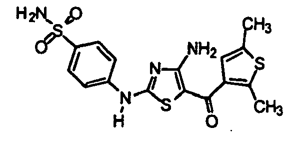
   3-Bmmoacetyl-2,5-dimethyl-thiophene, which has the structural formula 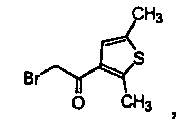 was prepared in a manner analogous to 2-bromo-2'-iodoacetophenone for Example C(12). 3-Acetyl-2, 5-dimethylthiophene (6.83 g, 44.3 mmol) provided 10.1 g (98% yield) of yellow oil, which was used without further purification.
   ¹HNMR (CDCl₃): δ 7.22 (1H, s), 4.64 (2H, s), 2.58 (3H, s), 2.36 (3H, s).
   The title compound was prepared in a manner analogous to that used in Example C(1). 4-Isothiocyanato-benzenesulfonamide and 3-bromoacetyl-2,5-dimethyl-thiophene provided a yellow powder in 69% yield, mp 263-5°C.
   ¹H NMR (DMSO-d₆): δ 11.02 (1H, s), 8.05 (2H, bs), 7.76 (4H, s), 7.25 (2H, s), 6.87 (1H, s), 2.43 (3H, s), 2.38 (3H, s).
   Anal. Calcd. for C₁₆H₁₆N₄O₃S₃: C, 47.04; H, 3.95; N, 13.71; S, 23.55. Found: C, 47.01; H, 3.92; N, 13.62; S, 23.47.
- Example C(51): 4-[4-Amino-5-(2-oxo-1,2,3,4-tetrahydro-quinoline-6-carbonyl)-thiazol-2-ylamino]-benzenesulfonamide 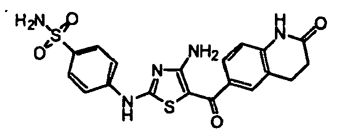
   The title compound was prepared essentially as described for Example C(1). 4-Isothiocyanato-benzenesulfonamide and 6-(bromoacetyl)-2-oxo-1,2,3,4-tetrahydroquinoline gave a grey-yellow solid in 48% yield, mp 300-305°C(d).
   ¹H NMR (DMSO-d₆): δ 11.08 (1H, s), 10.32 (1H, s), 8.17 (2H, bs), 7.82-7.70 (4H, m), 7.58-7.45 (3H, m), 7.27 (1H, s), 6.90 (1H, d, J = 8.1 Hz), 2.93 (4H, t, J = 7.7 Hz). IR (KBr): 3266, 3193, 3069, 1679, 1597, 1525, 1434, 1365, 1317, 1153 cm⁻¹. HRFABMS. Calcd. for C₁₉H₁₈N₅O₄S₂ (MH⁺): 444.0800. Found: 444.0816.
   Anal. Calcd for C₁₉H₁₇N₅O₄S₂ • 0.6 MeOH: C, 50.88; H, 4.23; N, 15.13; S, 13.86.
   Found: C, 51.02; H, 4.00; N, 15.00; S, 13.60.
- Example C(59): [4-Amino-2-(4-sulfamoyl-phenylamino)-thiazol-5-yl]-(3,5-dimethylpyridin-4-yl)-methanone 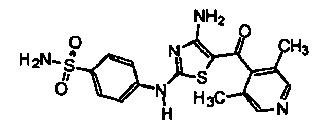
   4-(Bromoacetyl)-3,5-dimethylpyridine hydrobromide, which has the structural formula 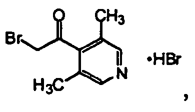 was first prepared as follows. 4-Acetyl-3,5-dimethylpyridine (500 mg, 3.36 mmol; Kutney et al., *Can. J. Chem*., vol. 41 (1963), pp. 695-702) was dissolved in 30% HBr in acetic acid (1 mL), heated to 70°C, and treated with a mixture of bromine (0.17 mL, 3.36 mmol) in 30% HBr in acetic acid (0.5 mL). After 2 hours, the mixture was allowed to cool to ambient temperature and ether (8 mL) was added. The resultant precipitate was filtered off, rinsed with ether (2x), and dried to afford 1.03 g (100%) of a purple solid, mp 222-225°C, that was used without further purification.
   The title compound was prepared essentially as described for Example C(1). 4-Isothiocyanato-benzenesulfonamide and 4-(bromoacetyl)-3,5-dimethylpyridine hydrobromide provided a tan solid, which was purified via column chromatography with 10% MeOH/CHCl₃ and crystallized from MeOH to obtain 35 mg (51%) of amorphous yellow solid.
   ¹H NMR (DMSO-d₆): δ 11.09 (1H, s), 8.32 (2H, s), 8.18 (2H, bs), 7.74 (4H, dd, J = 11.5,9.3 Hz), 7.27 (2H, s), 2.15 (6H, s).
   IR (KBr): 3378, 3342, 3260, 3160, 1625, 1594, 1560, 1518, 1443, 1342, 1160 cm⁻¹. HRFABMS: Calcd. for C₁₇H₁₈N₅O₃S₂ (MH⁺): 404.0851. Found: 404.0840.
   Anal. Calcd for C₁₇H₁₇N₅O₃S₂ • 0.4 H₂O • 0.3 MeOH: C, 49.44; H, 4.56; N, 16.66; S, 15.26. Found: C, 49.13; H, 4.31; N, 16.61; S, 15.10.
- Example C(65): 2S-[4-Amino-2-(4-sulfamoyl-phenylamino)-thiazole-5-carbonyl]-N-carbobenzyloxy-pyrrolidine 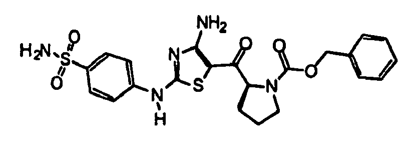
   The title compound was prepared essentially as described for Example C(1). 4-Isothiocyanato-benzenesulfonamide and 2S-bromoacetyl-N-carbobenzyloxy-pyrrolidine (see Example C(64)) provided a solid that was purified via column chromatography with 5% MeOH/CHCl₃ eluant to give a yellow amorphous solid, 140 mg (46%), mp 150-160° (d).
   ¹H NMR (DMSO-d₆): δ 11.05 (1H, d, J = 10.0 Hz), 7.98 (2H, bd, J = 17.1 Hz), 7.79 (4H, dd, J = 12.1, 9.7 Hz), 7.41-7.11 (5H, m), 5.15-4.89 (2H, m), 4.32-4.21 (1H, bm), 3.51-3.40 (2H, bm), 2.35-2.13 (1H, bm), 1.93-1.75 (3H, bm).
   IR (KBr): 3288, 1686, 1598, 1550, 1527, 1420, 1157 cm⁻¹.
   HRFABMS: Calcd. for C₂₂H₂₃N₅O₅S₂Cs (M+Cs⁺): 634.0195. Found: 634.0215.
   Anal. Calcd for C₂₂H₂₃N₅O₅S₂ • 0.3 H₂O • 0.1 CHCl₃: C, 51.15; H, 4.60; N, 13.50; S, 12.36. Found: C, 51.36; H, 4.63; N, 13.31; S, 12.47.
- Example C(73): 4-[4-Amino-5-(3,5-dibromo-thiophene-2-carbonyl)-thiazol-2-ylamino]-benzenesulfonamide 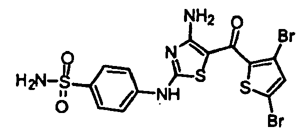
   The title compound was prepared in a manner analogous to that used in Example C(1). 4-Isothiocyanato-benzenesulfonamide and 2-bromoacetyl-3,5-dibromo-thiophene (from Example C(72)) provided a yellow powder in 41 % yield, mp 254-255°C.
   ¹HNMR (DMSO-d₆): δ 11.24 (1H, s), 8.31 (2H, bs), 7.77 (4H, s), 7.40 (1H, s), 7.28 (2H, s).
   FABMS (MH⁺): 536/538/540.
   Anal. Calcd. for C₁₄H₁₀N₄O₃S₃Br₂: C, 31.24; H, 1.87; N, 10.41; S, 17.87; Br, 29.69.
   Found: C, 31.08; H, 1.90; N, 10.16; S, 17.69; Br, 29.96.
- Example C(76): 4-[4-Amino-5-(1,5-dimethyl-1H-imidazole-4-carbonyl)-thiazol-2-ylamino]-benzenesulfonamide 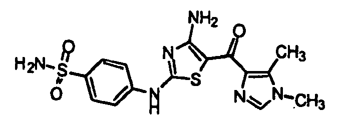
   The title compound was prepared in a manner analogous to that used in Example C(1). 4-Isothiocyanato-benzenesulfonamide and 5-bromoacetyl-1,5-dimethyl-1H-imidazole (from Example C(74)) provided a yellow solid in 8% yield, mp 293-294°C.
   ¹H NMR (DMSO-d₆): δ 10.80 (1H, s), 7.81 (2H, d, J = 9.0 Hz), 7.75 (2H, d, J = 9.0 Hz), 7.62 (1H, s), 7.24 (2H, s), 3.56 (3H, s), 2.52 (3H, s).
   HRFABMS (M+Na⁺): Caled.: 415.0623. Found: 415.0609.
   Anal. Calcd. for C₁₅H₁₆N₆O₃S₂ • 1.0 CH₃OH • 1.0 CHCl₃: C, 42.53; H, 4.45; N, 18.26; S, 13.93. Found: C, 42.57; H, 4.41; N, 18.18; S, 14.07.
- Example C(85): 4-[4-Amino-5-(2,6-difluoro-benzoyl)-thiazol-2-ylamino]-benzenesulfonamide 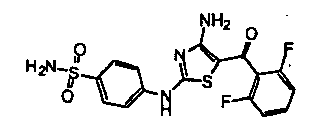
   The title compound was prepared in a manner analogous to that used in Example C(1). 4-Isothiocyanato-benzenesulfonamide and 2-bromo-2',6'-difluoro-acetophenone (from Example C(79)) provided light yellow crystals in 69% yield, mp 258-260°C.
   ¹H NMR (DMSO-d₆): δ 11.20 (1H, s), 8.20 (2H, bs), 7.79 (2H, d, J = 9.0 Hz), 7.74 (2H, d, J = 9.0 Hz), 7.61-7.49 (1H, m), 7.26 (2H, s), 7.22 (1H, d, J = 7.9 Hz), 7.19 (1H, d, J = 8.0 Hz).
   IR (KBr): 3310, 1622, 1599, 1547, 1525, 1467, 1425, 1410, 1318, 1156 cm⁻¹.
   HRFABMS (MH⁺): Calcd.: 411.0397. Found: 411.0410.
   Anal. Calcd. for C₁₆H₁₂N₄O₃S₂F₂ • 0.7 CH₃OH: C, 46.34; H, 3.45; N, 12.94; S, 14.82.
   Found: C, 46.19; H, 3.12; N, 12.83; S, 14.94.
- Example C(92): 4-[4-Amino-5-(2,5-dichloro-thiophene-3-carbonyl)-thiazol-2-ylamino]-benzenesulfonamide 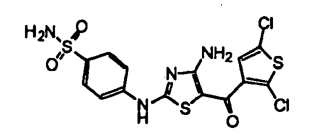
   3-Bromoacetyl-2,5-dichloro-thiophene, which has the structural formula 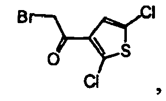 was prepared in a manner analogous to 2-bromo-2'-iodo-acetophenone, see Example C(12): 3-Acetyl-2,5-dichlorothiophene (2.0 g, 10.2 mmol) provided 2.9 g (100% yield) of yellow oil, which was used without further purification. ¹HNMR (CDCl₃): δ 7.25 (1H, s), 4.40 (2H, s).
   The title compound was prepared in a manner analogous to that used in Example C(1). 4-Isothiocyanato-benzenesulfonamide and 3-bromoacetyl-2,5-dichloro-thiophene provided a yellow solid in 65% yield, mp 274-276°C.
   ¹H NMR (DMSO-d₆): δ 11.20 (1H, s), 8.24 (2H, bs), 7.80 (4H, s), 7.33 (1H, s), 7.31 (2H, s).
   FABMS (MH⁺): 449/451.
   Anal. Calcd. for C₁₄H₁₀N₄O₃S₃Cl₂: C, 37.42; H, 2.24; N, 12.47; S, 21.41; Cl, 15.78.
   Found: C, 37.56; H, 2.19; N, 12.39; S, 21.29; Cl, 15.71.
- Example C(95): 4-[4-Amino-5-(3-methyl-5-nitro-thiophene-2-carbonyl)-thiazol-2-ylamino]-benzenesulfonamide 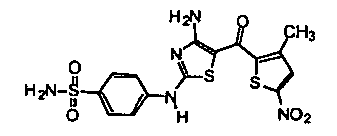
   The title compound was prepared in a manner analogous to that used in Example C(1). 4-Isothiocyanato-benzenesulfonamide and 2-bromoacetyl-3-methyl-5-nitrothiophene (from Example C(78)) provided a dark brown in 38% yield, mp 268-269°C.
   ¹H NMR (DMSO-d₆): δ 11.31 (1H, s), 8.46 (2H, bs), 8.08 (1H, s), 7.81 (4H, s), 7.32 (2H, s), 2.38 (3H, s).
   Anal. Calcd. for C₁₅H₁₃N₅O₅S₃: C, 40.99; H, 2.98; N, 15.94; S, 21.89. Found: C, 41.11; H,2.95; N, 15.66; S, 21.70.
- Example C(99): 4-[4-Amino-5-(2-fluoro-6-trifluommethyl-benzoyl)-thiazol-2-ylamino]-benzenesulfonamide 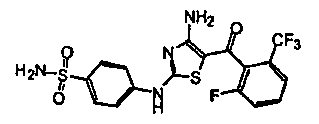
   2-Bromo-2'-fluoro-6'-trifluoromethyl-acetophenone, which has the structural formula 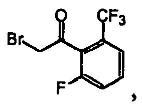 was prepared in a manner analogous to 2-bromo-2'-iodoacetophenone, see Example C(12). 2'-Fluoro-6'-(trifluoromethyl)-acetophenone (745 mg, 3.61 mmol) provided 1.05 g of yellow oil, which was used without further purification.
   ¹H NMR (CDCl₃): δ 7.69-7.52 (2H, m), 7.44-7.35 (1H, m), 4.42 (3H, s).
   The title compound was prepared in a manner analogous to that used in Example C(1). 4-Isothiocyanato-benzenesulfonamide and crude 2-bromo-2'-fluoro-6'-trifluoromethyl-acetophenone provided a light yellow solid in 21% yield, mp 290-292°C. ¹HNMR (DMSO-d₆): δ 11.15 (1H, s), 8.20 (2H, bs), 7.83-7.68 (7H, m), 7.31 (2H, s). Anal. Calcd. for C₁₇H₁₂N₄O₃S₂F₄: C, 44.35; H, 2.63; N, 12.17; S, 13.93. Found: C, 44.42; H, 2.64; N, 12.13; S, 13.94.
- Example C(107): 4-[4-Amino-5-(2,4,6-trichloro-benzoyl)-thiazol-2-yl-amino]-benzenesulfonamide 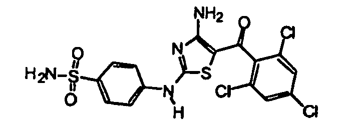
   2,4,6-Trichloroacetophenone, which has the structural formula 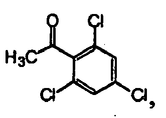 was first prepared as follows. Adapted from a procedure by Reynolds et al., *Org. Syn. Coll.,* vol. IV (1963), pp. 708-710. To Mg turnings (283 mg, 11.3 mmol) and EtOH (0.25 mL) was added CCl₄ (11 µL). The ensuing reaction subsided, before a solution of diethyl malonate (1.71 mL, 11.33 mmol) in EtOH (0.91 mL) was added at a rate to sustain reaction. After 30 min, the mixture was refluxed to consume Mg for one hour, then allowed to cool. The solid mass was suspended in ether (25 mL) and a solution of 2,4,6-trichlorobenzoyl chloride (2.50 g, 10.3 mL) in ether (5 mL) was added cautiously. After 3 days, a solution of H₂SO₄ (0.6 mL) in water (10 mL) was carefully added to dissolve any solids, and extracted with ether (2 x 10 mL). The extracts were dried over MgSO4 and evaporated to a cloudy oil, which was placed in HOAc (3 mL), H₂O (2 mL) and H₂SO₄ (0.33 mL), and heated to reflux. After 7.5 hours, the mixture was allowed to cool overnight. The mixture was made alkaline with 1N NaOH (35 mL) and extracted with ether (3 x 10 mL). The combined ethereal layers were dried with MgSO₄ and evaporated to give 1.80 g (78%) of a white solid that was used without further purification (previously described in Baker et al., *J. Chem. Soc.* (1941), pp. 796-802).
   2-Bromo-2',4',6'-trichloroacetophenone, which has the structural formula 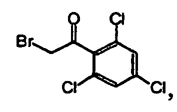 was prepared in a manner analogous to 2-bromo-2'iodo-acetophenone for Example C(12). Crude 2',4',6'-trichloroacetophenone afforded 1.27 g (94%) of gold crystals that were used without further purification (previously described in Baker et al., J. Chem. Soc. (1941), pp. 796-802).
   ¹H NMR: δ 7.42 (2H, s), 4.42 (s, 2H).
   The title compound was prepared essentially as described for Example C(1), except that excess potassium t-butoxide (2.2 equivalents) was employed. 4-Isothiocyanato-benzenesulfonamide and 2-bromo-2',4',6'-trichloroacetophenone provided a dark brown gum, which was purified via column chromatography with 10% MeOH/CHCl₃ and precipitated from MeOH/CHCl₃ to obtain 96 mg (21 %) of an amorphous, pale yellow solid.
   ¹H NMR (CD₃OD): δ 7.87 (4H, dd, J = 14.6, 9.0 Hz), 7.60 (2H, s).
   IR (KBr): 3312, 1593, 1545, 1459, 1421, 1161 cm⁻¹.
   ESIMS (MH⁺): 477/ 479/481. (M⁻): 475/477/479.
   Anal. Calcd for C₁₆H₁₁Cl₃N₄O₃S₂: C, 40.22; H, 2.32; N, 11.73; Cl, 22.26; S, 13.42. Found: C, 40.12; H, 2.34; N, 11.56; Cl, 22.41; S, 13.43.
- Example C(108): 4-[4-Amino-5-(2,6-difluoro-benzoyl)-thiazol-2-ylamino]-N-methyl-benzenesulfonamide 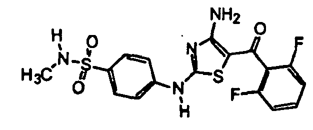
   4-Amino-N-methyl-benzenesulfonamide, which has the structural formula 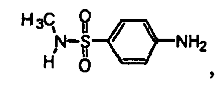 was first made as follows. N-Methyl-4-nitro-benzenesulfonamide (2.58 g, 11.9 mmol; Khanna et al., *J. Med. Chem*., vol. 40 (1997), pp. 1619-1633) and 10% Pd/C (250 mg) in MeOH (60 mL) was stirred under hydrogen atmosphere for 2 hours and filtered. The filtrate was concentrated *in vacuo* to provide 2.17 g (98% yield) of colorless crystalline flakes, which by ¹H NMR matched that reported in the literature (Khanna et al., *J. Med. Chem*., vol. 40 (1997), pp. 1619-1633) and was used without further purification.
   4-Isothiocyanato-N-methyl-benzenesulfonamide, which has the structural formula 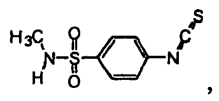 was prepared in a manner analogous to 4-isothiocyanato-benzamide of Example C(102). 4-Amino-N-methyl-benzenesulfonamide (2.17 g, 11.7 mmol) gave 2.10 g (79% yield) of white fluffy powder, which was used without further purification. ¹HNMR (DMSO-d₆): δ 7.83 (2H, d, J = 8.4 Hz), 7.65 (2H, d, J = 8.4 Hz), 7.61 (1H, q, J = 4.9 Hz), 2.43 (3H, d, J = 4.9 Hz).
   The title compound was prepared in a manner analogous to that used in Example C(1). 4-Isothiocyanato-N-methyl-benzenesulfonamide and 2-bromo-2',6'-difluoroacetophenone (from Example C(79)) provided a crude product, which was extracted with 10% i-PrOH/CHCl₃ and purified via column chromatography with 5% MeOH/CHCl₃ to afford an amorphous yellow powder in 41 % yield, that decomposed above 200°C.
   ¹HNMR (DMSO-d₆): δ 11.23 (1H, s), 8.33 (2H, bs), 7.81 (2H, d, J = 8.5 Hz), 7.54 (2H, d, J = 8.5 Hz), 7.63-7.41 (1H, m), 7.39 (1H, q, J = 5.0 Hz), 7.23 (2H, t, J = 7.1 Hz), 2.41 (3H, d, J = 5.0 Hz).
   HRFABMS (MH⁺): Calcd.: 425.0554. Found: 425.0566
   Anal. Calcd. for C₁₇H₁₄N₄O₃S₂F₂ • 0.5 CH₃OH: C, 47.72; H, 3.66; N, 12.72; S, 14.56.
   Found C, 47.56; H, 3.52; N, 12.72; S, 14.77.
- Example C(109): 4-[4-Amino-5-(2,6-difluoro-benzoyl)-thiazol-2-ylamino]-N,N-dimethyl-benzenesulfonamide 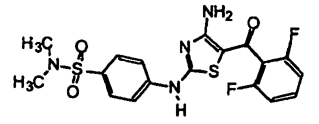
   4-Amino-N, N-dimethyl-benzenesulfonamide, which has the structural formula 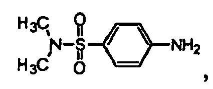 was next prepared as follows. Crude N, N-dimethyl-4-nitrobenzenesulfonamide (3.89 g, 16.9 mmol; Khanna et al., *J. Med. Chem.,* vol. 40 (1997), pp. 1619-1633), 10% Pd/C (800 mg), MeOH (80 mL), and THF (200 mL) were stirred under hydrogen for 6 hours and filtered. The filtrate was concentrated *in vacuo* to furnish 3.68 g of yellow solid, which was identical by ¹H NMR spectrum to previous description by Khanna et al., *J. Med. Chem*., vol. 40 (1997), pp. 1619-1633 and was used without further purification.
   4-Isothiocyanato-N, N-dimethyl-benzenesulfonamide, which has the structural formula 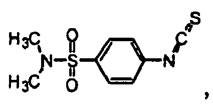 was next made as follows. To a solution of 4-amino-N, N-dimethyl-benzenesulfonamide (2.0 g, 10 mmol) in acetone (50 mL) at 5-10°C were added simultaneously a solution of thiophosgene (0.91 mL, 12 mmol) in acetone (20 mL) and 25% aq Na₂CO₃ (10 mL). After 5 min at 5-8°C, the mixture was allowed to warm and was stirred at ambient temperature for a half hour. The solvent was evaporated and water (70 mL) was added. The resultant light-yellow precipitate was filtered off, washed with water, and dried under vacuum to afford 2.35 g (97% yield) of white powder, which was used without further purification.
   ¹H NMR (DMSO-d₆): δ 7.82 (2H, d, J = 8.4 Hz), 7.69 (2H, d, J = 8.4 Hz), 2.63 (6H, s).
   The title compound was prepared in a manner analogous to that used in Example C(1). 4-Isothiocyanato-N, N-dimethyl-benzenesulfonamide and 2-bromo-2',6'-difluoroacetophenone (from Example C(79)) provided a crude brown solid that recrystallized from EtOH to give light-brown crystals in 52% yield, mp 240-242°C.
   ¹H NMR (DMSO-d₆): δ 11.24 (1H, s), 8.14 (2H, bs), 7.84 (2H, d, J = 8.8 Hz), 7.72 (2H, d, J = 8.8 Hz), 7.62-7.49 (1H, m), 7.23 (1H, d, J = 7.9 Hz), 7.20 (1H, d, J = 8.0 Hz), 2.59 (6H, s).
   Anal. Calcd. for C₁₈H₁₆N₄O₃S₂F₂: C, 49.31; H, 3.68; N, 12.78; S, 14.63. Found: C, 49.29; H, 3.71; N, 12.68; S, 14.50.
- Example C(114): 4-[4-Amino-5-(3-methyl-thiophene-2-carbonyl)-thiazol-2-ylamino]-N-methyl-benzenesulfonamide 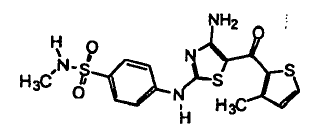
   The title compound was prepared in a manner analogous to that used in Example C(1). 4-Isothiocyanato-N-methyl-benzenesulfonamide (from Example C(108)) and 2-bromoacetyl-3-methyl-thiophene (from Example C(19)) provided a yellow solid in 57% yield, mp 197.0-199.5°C.
   ¹H NMR (DMSO-d₆): δ 11.19 (1H, s), 8.24 (2H, bs), 7.86 (2H, d, J = 8.7 Hz), 7.75 (2H, d, J = 8.7 Hz), 7.65 (1H, d, J = 5.0 Hz), 7.36 (1H, q, J = 6.1 Hz), 7.03 (1H, d, J = 5.0 Hz), 2.42 (3H, S), 2.41 (3H, d, J = 6.1 Hz).
   HRFABMS (MH⁺): Calcd.: 409.0463. Found: 409.0474.
   Anal. Calcd. for C₁₆H₁₆N₄O₃S₃ • 0.4 H₂O: C, 46.23; H, 4.07; N, 13.48; S, 23.14. Found: C, 46.28; H, 3.98; N, 13.38; S, 23.08.
- Example C(115): 4-[4-Amino-5-(2,4,6-trifluoro-benzoyl)-thiazol-2-yl-amino]-benzenesulfonamide 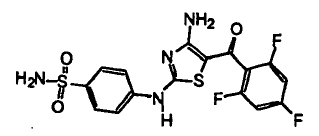
   2-Chloro-2',4',6'-trifluoroacetophenone, which has the structural formula 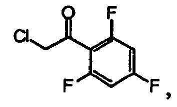 was first prepared as follows. To a mechanically stirred solution of 1,3,5-trifluorobenzene (5.17 mL, 50.0 mmol) in dichloroethane (12.5 mL) was added gradually AlCl₃ (13.4 g, 115 mmol) over 15 min. time period with caution. Violent bumping and HCl gas evolution was observed. The mixture was carefully heated to reflux, and chloroacetyl chloride (6.20 g, 4.37 mL, 55.0 mmol) was added dropwise over 45 min. time period. After 6 hours at reflux, the mixture was allowed to cool over 12 hours, then carefully poured onto an ice/water slush (~200 mL) and extracted with ether (3 x 50 mL). The combined ethereal layers were washed with 10% aq. HCl (2 x 30 mL), 1N aq. NaOH (3 x 30 mL), and brine (25 mL), dried over MgSO4 and evaporated to give 5.28 g (51%) of a yellow solid that was used without further purification. (An analytical sample crystallized from ether/hexane to give yellow microcrystals, mp 43-45°C.)
   ¹H NMR (CDCl₃): δ 6.81 (2H, t, J = 8.4 Hz), 4.54 (2H, s).
   IR (KBr): 1721, 1637, 1616, 1447, 1201, 1128, 1045 cm⁻¹.
   Anal. Calcd. for C₈H₄ClF₃O: C, 46.07; H, 1.93; Cl, 17.00. Found: C, 45.92; H, 1.95; Cl, 16.97.
   The title compound was prepared essentially as described for Example C(1), except that excess potassium t-butoxide (2.2 equivalents) was employed. 4-Isothiocyanato-benzenesulfonamide and 2-chloro-2',4',6'-trifluoroacetophenone gave a red-brown solid, which was purified via column chromatography with 5% MeOH/CH₂Cl₂ as eluant. Precipitation with trace hexane in MeOH/CH₂Cl₂ gave 70 mg (33%) of yellow amorphous powder that decomposed above 148°C.
   ¹H NMR (CD₃OD): δ 7.91 (1H, s), 7.86 (4H, dd, J =14.9, 6.9 Hz), 6.99 (2H, dd, J = 9.0, 7.5 Hz).
   IR (KBr): 3278, 1602, 1549, 1425, 1155 cm⁻¹.
   HRFABMS. Calcd for C₁₆H₁₂F₃N₄O₃S₂ (MH⁺): 429.0303. Found: 429.0315.
   Anal. Calcd for C₁₆H₁₁F₃N₄O₃S₂ • 1.1 H₂O: C, 42.87; H, 2.97; N, 12.50; S, 14.31. Found: C, 42.98; H, 2.73; N, 12.12; S, 14.48.
- Example C(116): {4-Amino-2-[4-(4-methyl-piperazine-1-sulfonyl)-phenylamino]-thiazol-5-yl}-(2,6-difluoro-phenyl)-methanone 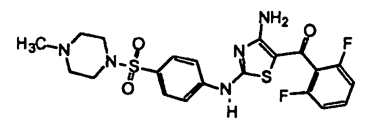
   1-Methyl-4-(4-nitro-benzenesulfonyl)-piperazine, which has the structural formula 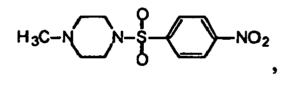 was prepared in a manner analogous to that used for N-methyl-4-nitro-benzenesulfonamide for Example C(108) (Khanna et al., *J. Med. Chem.,* vol. 40 (1997), pp. 1619-1633). 4-Nitrobenzenesulfonyl chloride and 1-methylpiperazine gave 5.1 g (88% yield) of yellow solid, which was used without further purification.
   4-(4-Methyl-piperazine-1-sulfonyl)-aniline, which has the structural formula 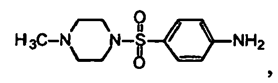 was prepared in a manner analogous to that used for N-methyl-4-amino-benzenesulfonamide for Example C(108). 1-Methyl-4-(4-nitrobenzenesulfonyl)-piperazine provided a gray solid in 99% yield, which was used in the next step without further purification.
   ¹H NMR (DMSO-d₆): δ 7.37 (2H, d, J = 8.8 Hz), 6.67 (2H, d, J = 8.8 Hz), 6.16 (2H, bs), 3.30 (4H, bs), 3.03 (4H, bs), 2.58 (3H, s).
   1-(4-Isothiocyanato-benzenesulfonyl)-4-methyl-piperazine, which has the structural formula 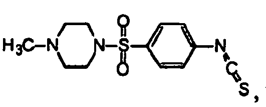 was made in a manner analogous to 4-isothiocyanato-benzamide for Example C(102). 4-(4-Methyl-piperazine-1-sulfonyl)-aniline provided 1.1 g (94% yield) of white crystals which were used without further purification.
   ¹HNMR (CDCl₃): δ 7.74 (2H, d, J = 8.6 Hz), 7.35 (2H, d, J = 8.6 Hz), 3.27 (4H, bs), 2.77 (4H, bs), 2.47 (3H, s).
   The title compound was prepared in a manner analogous to that used in Example C(1). 1-(4-Isothiocyanato-benzenesulfonyl)-4-methyl-piperazine and 2-bromo-2',6'-difluoro-acetophenone (from Example C(79)) provided a yellow solid in 69% yield, mp 172-174°C.
   ¹H NMR (DMSO-d₆): δ 11.23 (1H, bs), 8.21 (2H, bs), 7.84 (2H, d, J = 8.8 Hz), 7.69 (2H, d, J = 8.8 Hz), 7.62-7.49 (1H, m), 7.22 (1H, d, J = 7.8 Hz), 7.19 (1H, d, J = 8.1 Hz), 2.87 (4H, t, J = 4.5 Hz), 2.35 (4H, t, J = 4.5 Hz), 2.13 (3H, s).
   HRFABMS (MH⁺): Calcd.: 494.1132. Found: 494.1120.
   Anal. Calcd. for C₂₁H₂₁N₅O₃S₂F₂ • 0.1 H₂O • 0.5 CH₃OH: C, 50.50; H, 4.57; N, 13.70; S, 12.54. Found: C, 50.34; H, 4.39; N, 13.51; S, 12.63.
- Example D(5): 4-[4-Amino-5-(3-amino-5-amino-thiophene-2-carbonyl)-thiazol-2-ylamino]-benzenesulfonamide 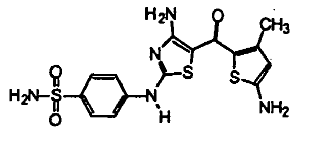
   The title compound was prepared in a manner analogous to that used in Example D(1). The title compound of Example C(95) was hydrogenated and recrystallized from EtOH to provide a brown powder in 96% yield, mp 268-271°C.
   ¹H NMR (DMSO-d₆): δ 10.97 (1H, s), 7.91 (2H, s), 7.82 (2H, d, J = 9.1 Hz), 7.78 (2H, d, J = 9.1 Hz), 7.28 (2H, s), 6.43 (2H, s), 5.81 (1H, s), 2.34 (3H, s).
   FABMS (MH⁺): 410.
   Anal. Calcd. for C₁₅H₁₅N₅O₃S₃ • 0.1 H₂O • 0.3 EtOH: C, 44.07; H, 4.03; N, 16.47; S, 22.63. Found: C, 44.23; H, 3.93; N, 16.07; S, 23.01.
- Example E(2): 4-[4-Amino-2-(4-sulfamoyl-phenylamino)-thiazole-5-carbonyl]-benzoic Acid 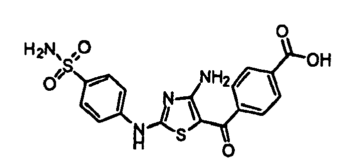
   To a suspension of ethyl 4-[4-amino-2-(4-sulfamoyl-phenylamino)-thiazole-5-carbonyl]-benzoate (500 mg, 1.12 mmol; Example C(34)) in MeOH (10 mL) was added 1N aq NaOH (3.4 mL, 3.4 mmol). After 4 hours, the resultant mixture was acidified with 1N aq HCl to pH 3 and filtered. The isolated brown solid crystallized in EtOH to provide 330 mg (70% yield) of light brown crystals, mp 298.5-300°C.
   ¹H NMR (DMSO-d₆): δ 13.15 (1H, s), 11.14 (1H, s), 8.31 (2H, bs), 8.02 (2H, d, J = 8.1 Hz), 7.78 (4H, s ), 7.77 (2H, d, J = 8.1 Hz), 7.26 ( 2H, s).
   HRFABMS (M+Na⁺): Calcd.: 441.0303. Found: 441.0320.
   Anal. Calcd. for C₁₇H₁₄N₄O₅S₂ • 0.4 H₂O: C, 47.97; H, 3.50; N, 13.16; S, 15.07. Found: C, 48.04; H, 3.48; N, 12.98; S, 15.18.
- Example J(4): 4-[4-Amino-5-(2,6-difluoro-benzoyl)-thiazol-2-ylamino]-N-piperidin-4-ylmethyl-benzenesulfonamide 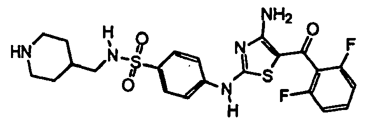
   N-tert-Butoxycarbonyl-4-carbamoyl-piperidine, which has the structural formula 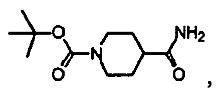 was made as follows. To isonipecotamide (5.00 g, 39.0 mmol) in dioxane (100 mL) was added di-tert-butyl dicarbonate (8.51 g, 39.0 mmol) and N, N-diisopropylethylamine (6.0 mL, 42.9 mmol). The mixture was allowed to stir overnight, then evaporated undr reduced pressure to dryness. The residue was partitioned between CHCl₃ and 1N HCl. The organic layer was washed with water and brine, dried over Na₂SO₄, and concentrated to give 8.3 g (93% yield) of white solid, which was used without further purification.
   ¹H NMR (CDCl₃): δ 5.53 (2H, bs), 4.03 (2H, d, J = 13.7 Hz), 2.33 (2H, tt, J = 11.8, 3.7 Hz), 2.08 (2H, bs), 1.89 (2H, dd, J = 13.7, 3.7 Hz), 1.69 (1H, dd, J = 11.8, 4.4 Hz), 1.65-1.57 (1H, m), 1.44 (9H, s).
   4-Aminomethyl-N-tert-butoxycarbonyl-piperidine, which has the structural formula 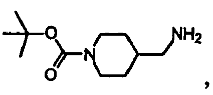 was made as follows. To N-tert-butoxycarbonyl-4-carbamoyl-piperidine (15.6 mmol) in THF (40 mL) at -78°C under Ar was added LiAlH₄ (592 mg, 15.6 mmol). The mixture was allowed to warm to ambient temperature slowly and after a half hour, recooled to -78°C, quenched with ethyl acetate, and partitioned between EtOAc and 2N NaOH. The organic layer was separated, dried over K₂CO₃, and concentrated to give 1.98 g (59% yield) of yellow slurry, which was used without further purification.
   N-tert-Butoxycarbonyl-4-[(4-nitro-benzenesulfonylamino)-methyl]-piperidine, which has the structural formula 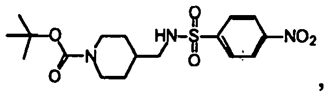 was made as follows. 4-nitrobenzenesulfonyl chloride (2.05 g, 9.24 mmol) was added to a solution of 4-aminomethyl-N-tert-butoxycarbonyl-piperidine (1.98 g, 9.24 mmol) in THF (20 mL) at ambient temperature. The mixture was refluxed for 1 hour, concentrated *in vacuo,* and partitioned between CH₂Cl₂ and 1N HCl. The organic layer was washed with brine, dried over Na₂SO₄, passed through a pad of silica gel, and concentrated to give 1.71 g (46% yield) of yellow solid, which was used without further purification.
   4-[(4-Amino-benzenesulfonylamino)-methyl]-N-tert-butoxycarbonyl-piperidine, which has the structural formula 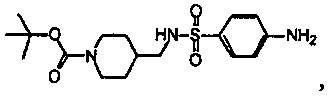 was prepared as follows. N-tert-Butoxycarbonyl-4-[(4-nitro-benzenesulfonylamino)-methyl]-piperidine (1.70 g, 4.26 mmol), 10 % Pd/C (250 mg), MeOH (10 mL), and THF (10 mL) was stirred under hydrogen for 2 hours and filtered. The filtrate was concentrated to a residue that was purified via column chromatography with 5% MeOH/CHCl₃ as eluant, producing 1.39 g (88% yield) of white solid, which was used without further purification.
   N-tert-Butoxycarbonyl-4-[(4-isothiocyanato-benzenesulfonylamino)-methyl]-piperidine, which has the structural formula 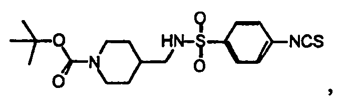 was prepared in a manner analogous to 1-(4-isothiocyanato-phenyl)-morpholine for Example C(54). 4-[(4-Amino-benzenesulfonylamino)-methyl]-N-tert-butoxycarbonyl-piperidine provided a yellow solid in 39% yield, which was used without further purification.
   4- {[4-(5-Acetyl-4-amino-thiazol-2-ylamino)-benzenesulfonylamino]-methyl}-N-tert-butoxycarbonyl-piperidine, which has the structural formula 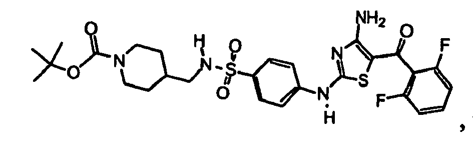 was prepared in a manner analogous to that used in Example C(1). N-tert-Butoxycarbonyl-4-[(4-isothiocyanatobenzenesulfonylamino)-methyl]-piperidine and 2-bromo-2',6'-difluoro-acetophenone (from Example C(79)) provided a yellow solid in 50% yield.
   ¹HNMR (DMSO-d₆): δ 11.22 (1H, s), 8.20 (2H, bs), 7.84-7.73 (3H, m), 7.62-7.54 (2H, m), 7.24 (2H, dd, J = 7.8, 7.7 Hz), 3.89 (2H, d, J =12.8 Hz), 3.35 (2H, s), 2.52 (2H, d, J = 1.2 Hz), 1.60 (2H, d, J = 10.1 Hz), 1.56-1.42 (1H, m), 1.39 (9H, s), 0.91 (2H, d, J = 12.8 Hz).
   The title compound was prepared in a manner analogous to that used in Example J(1). 4-{[4-(5-Acetyl-4-amino-thiazol-2-ylamino)-benzenesulfonylamino]-methyl}-N-tert-butoxycarbonyl-piperidine provided a brown solid in 28% yield.
   ¹HNMR (DMSO-d₆): δ 8.11 (2H, bs), 7.70 (4H, bs), 7.58-7.42 (1H, m), 7.20 (1H, d, J = 7.8 Hz), 7.15 (1H, d, J = 7.8 Hz), 3.80 (2H, bs), 3.05 (2H, d, J = 10.0 Hz), 2.60 (2H, d, J = 6.8 Hz), 1.65 (2H, d, J = 12.2 Hz), 1.52 (1H, bs), 1.07 (2H, d, J = 10.0 Hz).
   HRFABMS (MH⁺): Calcd.: 507.1210. Found: 507.1206.
   Anal. Calcd. for C₂₂H₂₃N₅O₃S₂F₂ • 0.1CH₃OH • 0.2 CF₃COOH: C, 50.65; H, 4.73; N, 13.12; S, 12.02. Found: C, 50.92; H, 4.46; N, 12.87; S, 12.18.
   Other compounds may be made in accordance with the invention in manners similar to those described above. Additional exemplary compounds of the invention are identified in Tables I, II, and III below, which provide results of biochemical and biological assays.

### BIOCHEMICAL AND BIOLOGICAL EVALUATION:

Cyclin-dependent kinase activity was measured by quantifying the enzyme-catalyzed, time-dependent incorporation of radioactive phosphate from [³²P]ATP or [³³P]ATP into a protein substrate. Unless noted otherwise, assays were performed in 96-well plates in a total volume of 50 µL, in the presence of 10 mM HEPES (N-[2-hydroxyethyl]piperazine-N'-[2-ethanesulfonic acid]) (pH 7.4), 10 mM MgCl₂, 25 µM adenosine triphosphate (ATP), 1 mg/mL ovalbumin, 5 µg/mL leupeptin, 1 mM dithiothreitol, 10 mM β-glycerophosphate, 0.1 mM sodium vanadate, 1 mM sodium fluoride, 2.5 mM ethylene glycol-bis(β-aminoethyl ether)-N,N,N'N'-tetraacetic acid (EGTA), 2% (v/v) dimethylsulfoxide, and 0.03 - 0.4 µCi [^{32/33}P]ATP per reaction. Reactions were initiated with enzyme, incubated at 30°C, and terminated after 20 minutes by the addition of ethylenediaminetetraacetic acid (EDTA) to 250 mM. The phosphorylated substrate was then captured on a nitrocellulose or phosphocellulose membrane using a 96-well filtration manifold, and unincorporated radioactivity was removed by repeated washing with 0.85% phosphoric acid. Radioactivity was quantified by exposing the dried membranes to a phosphorimager.

Apparent Kᵢ values were measured by assaying enzyme activity in the presence of different inhibitor compound concentrations and subtracting the background radioactivity measured in the absence of enzyme. The kinetic parameters (kcat, Km for ATP) were measured for each enzyme under the usual assay conditions by determining the dependence of initial rates on ATP concentration. Inhibition data were fit to an equation for competitive inhibition using Kaleidagraph (Synergy Software), or were fit to an equation for competitive tight-binding inhibition using the software KineTic (BioKin, Ltd.).

### Inhibition of CDK4/Cyclin D Retinoblastoma Kinase Activity:

A complex of human CDK4 and cyclin D3, or a complex of cyclin D1 and a fusion protein of human CDK4 and gluathione-S-transferase (GST-CDK4), or a complex of human CDK4 and genetically truncated (1-264) cyclin D3, was purified using traditional biochemical chromatographic techniques from insect cells that had been co-infected with the corresponding baculovirus expression vectors (see e.g., Meijer and Kim, "Chemical Inhibitors of Cyclin-Dependent Kinases," *Methods in Enzymol,.* vol. 283 (1997), pp. 113-128.). The enzyme complex (5 or 50 nM) was assayed with 0.3-0.5 µg of purified recombinant retinoblastoma protein fragment (Rb) as a substrate. The engineered Rb fragment (residues 386-928 of the native retinoblastoma protein; 62.3 kDa) contains the majority of the phosphorylation sites found in the native 106-kDa protein, as well as a tag of six histidine residues for ease of purification. Phosphorylated Rb substrate was captured by microfiltration on a nitrocellulose membrane and quantified using a phosphorimager as described above. For measurement of tight-binding inhibitors, the enzyme complex concentration was lowered to 5 nM, and the assay duration was extended to 60 minutes, during which the time-dependence of product formation was linear.

### Inhibition of CDK2/Cyclin A Retinoblastoma Kinase Activity:

CDK2 was purified using published methodology (Rosenblatt et al., "Purification and Crystallization of Human Cyclin-dependent Kinase 2," *J. Mol. Biol.,* vol. 230, 1993, pp. 1317-1319) from insect cells that had been infected with a baculovirus expression vector. Cyclin A was purified from *E. coli* cells expressing full-length recombinant cyclin A, and a truncated cyclin A construct was generated by limited proteolysis and purified as described previously (Jeffrey et al., "Mechanism of CDK activation revealed by the structure of a cyclin A-CDK2 complex," *Nature,* vol. 376 (27 July 1995), pp. 313-320). Purified, proteolyzed cyclin A was included in the assay at a three- to five-fold molar excess to CDK2. Alternatively, a complex of CDK2 and proteolyzed cyclin A was prepared and purified by gel filtration. The substrate for this assay was the same Rb substrate fragment used for the CDK4 assays, and the methodology of the CDK2/cyclin A and the CDK4/cyclin D3 assays was essentially the same, except that CDK2 was present at 150 nM or 5 nM. Kᵢ values were measured as described above.

### Inhibition of CDK1(cdc2)/Cyclin B Histone H1 Kinase Activity:

The complex of human CDK1 (cdc2) and cyclin B was purchased from New England Biolabs (Beverly MA). Alternatively, a CDK1/glutathione-S-transferase-cyclin B1 complex was purified using glutathione affinity chromatography from insect cells that had been co-infected with the corresponding baculovirus expression vectors. The assay was executed as described above at 30°C using 2.5 units of cdc2/cyclin B, 10 µg Histone H1 protein, and 0.1-0.3 µCi [^{32/33}P]ATP per assay. Phosphorylated histone substrate was captured by microfiltration on a phosphocellulose P81 membrane and quantified using a phosphorimager as described above. Kᵢ values were measured using the described curve-fitting programs.

Results of assays performed on compounds, which include the specific examples described above as well as additional examples designated by the prefix "I" (e.g., Examples I(1), I(2), etc.), where "*" denotes a compound having a known structure (i.e., the compound *per se* is known), are provided below in Tables I, II, and III. Unless indicated otherwise in a particular entry, the units and assays used are as indicated in the applicable column of the table. The abbreviation "N.I." indicates that no inhibition was observed at the concentration indicated.

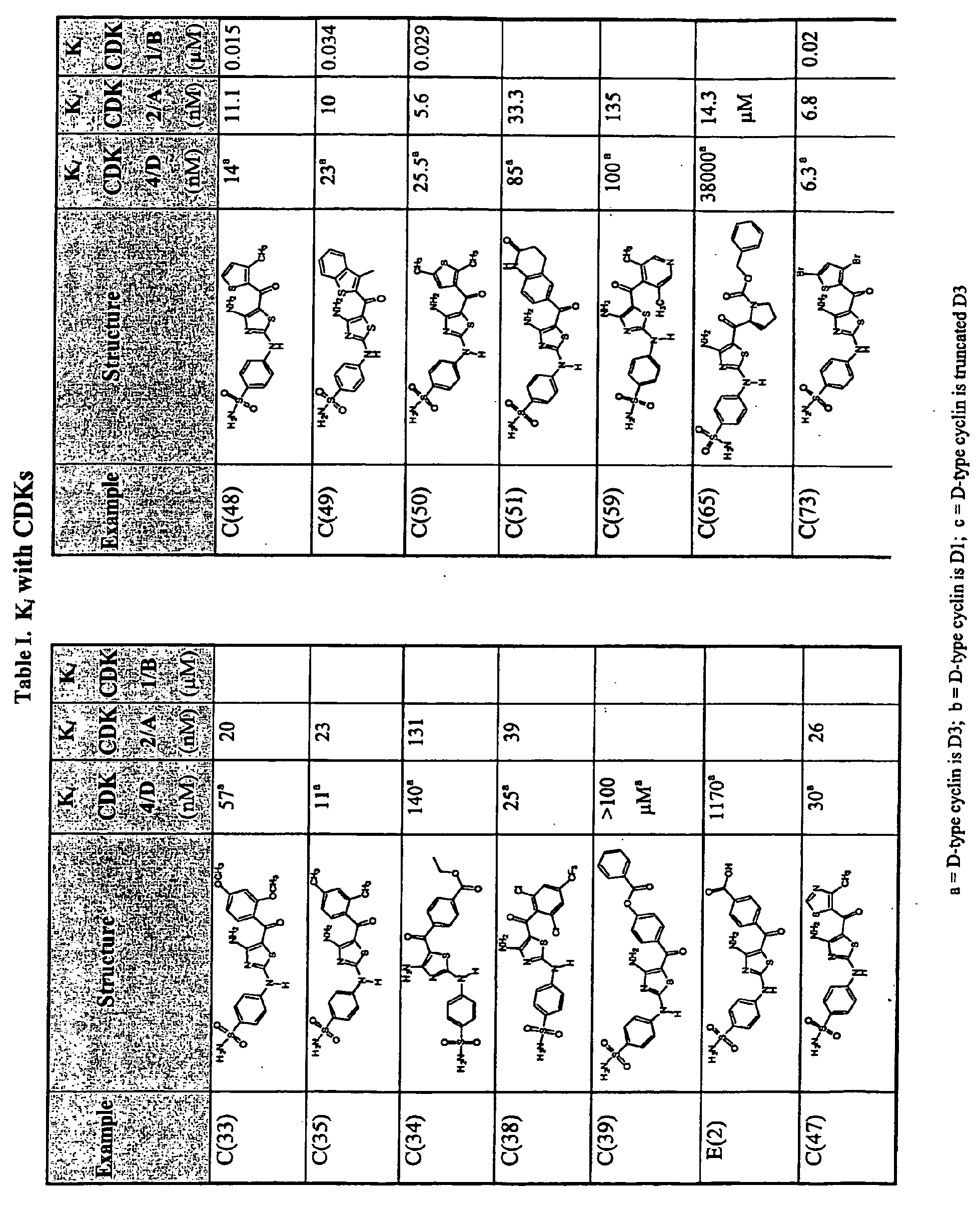

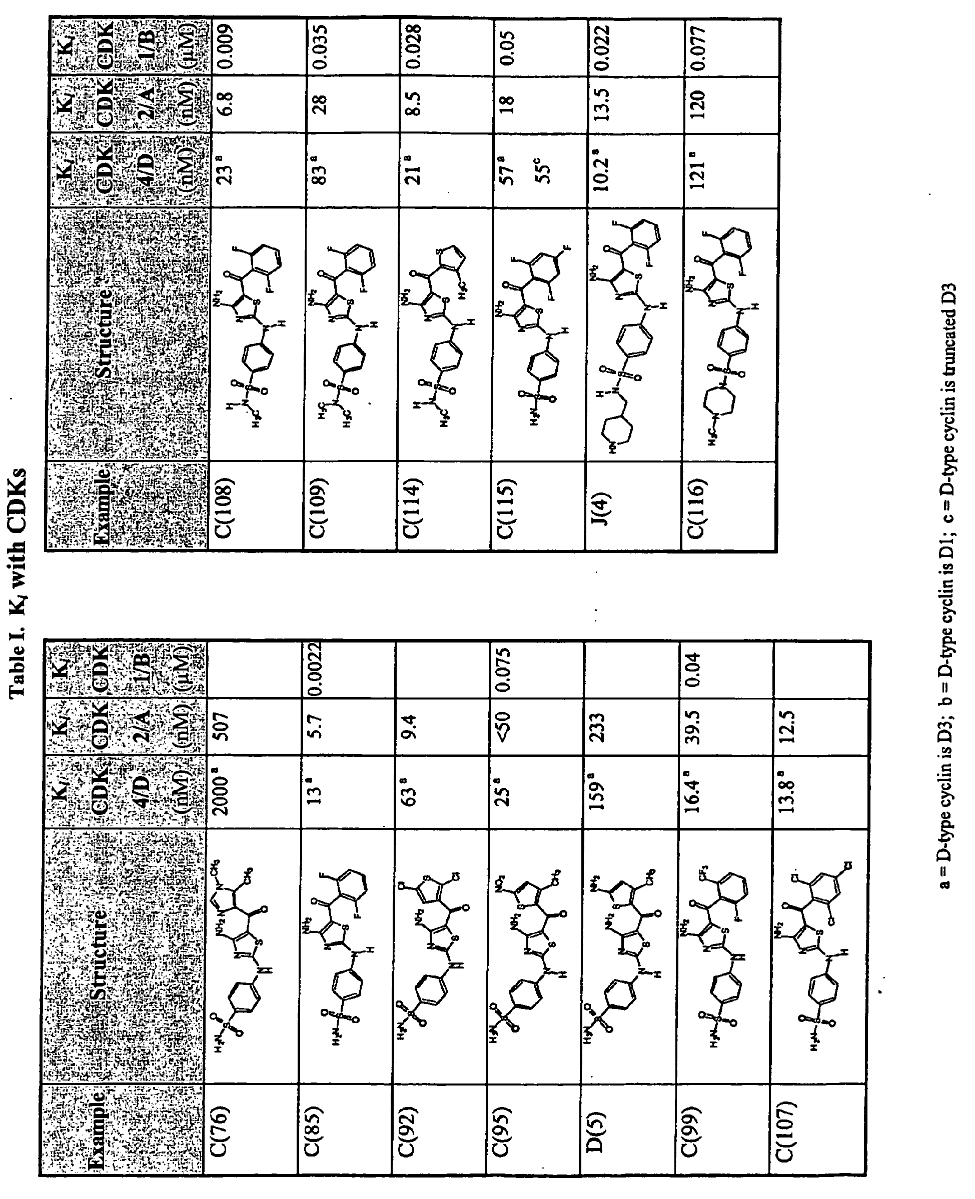

### Inhibition of Cell Growth: Assessment of Cytotoxicity:

Inhibition of cell growth was measured using the tetrazolium salt assay, which is based on the ability of viable cells to reduce 3-(4,5-dimethylthiazol-2-yl)-2,5-[2H]-diphenyltetrazolium bromide (MTT) to formazan (Mossman, *Journal of Immunological Methods,* vol. 65 (1983), pp. 55-58). The water-insoluble purple formazan product was then detected spectrophotometrically. Various cell lines (HCT-116, Saos-2, U2-OS, SW480, COLO-205, RXF-393, M14, MDA-MB-468, and MCF7) were grown in 96-well plates. Cells were plated in the appropriate medium at a volume of 135 µl/well in either McCoy's 5A Medium (for Saos-2, U2-OS, SW480, and HCT-116 cells), RPMI (for COLO-205, RXF-393, M14 cells), or Minimum Essential Medium Eagle (for MDA-MB-468 and MCF7 cells). Plates were incubated for four hours before addition of inhibitor compounds. Different concentrations of inhibitor compounds were added in 0.5% (v/v) dimethylsulfoxide (15 µL/well), and cells were incubated at 37°C (5% CO₂) for four to six days (depending on cell type). At the end of the incubation, MTT was added to a final concentration of 0.2 mg/mL, and cells were incubated for 4 hours more at 37°C. After centrifugation of the plates and removal of medium, the absorbance of the formazan (solubilized in dimethylsulfoxide) was measured at 540 nm. The concentration of inhibitor compound causing 50% inhibition of growth was determined from the linear portion of a semi-log plot of inhibitor concentration versus percentage inhibition. All results were compared to control cells treated only with 0.5% (v/v) dimethylsulfoxide.

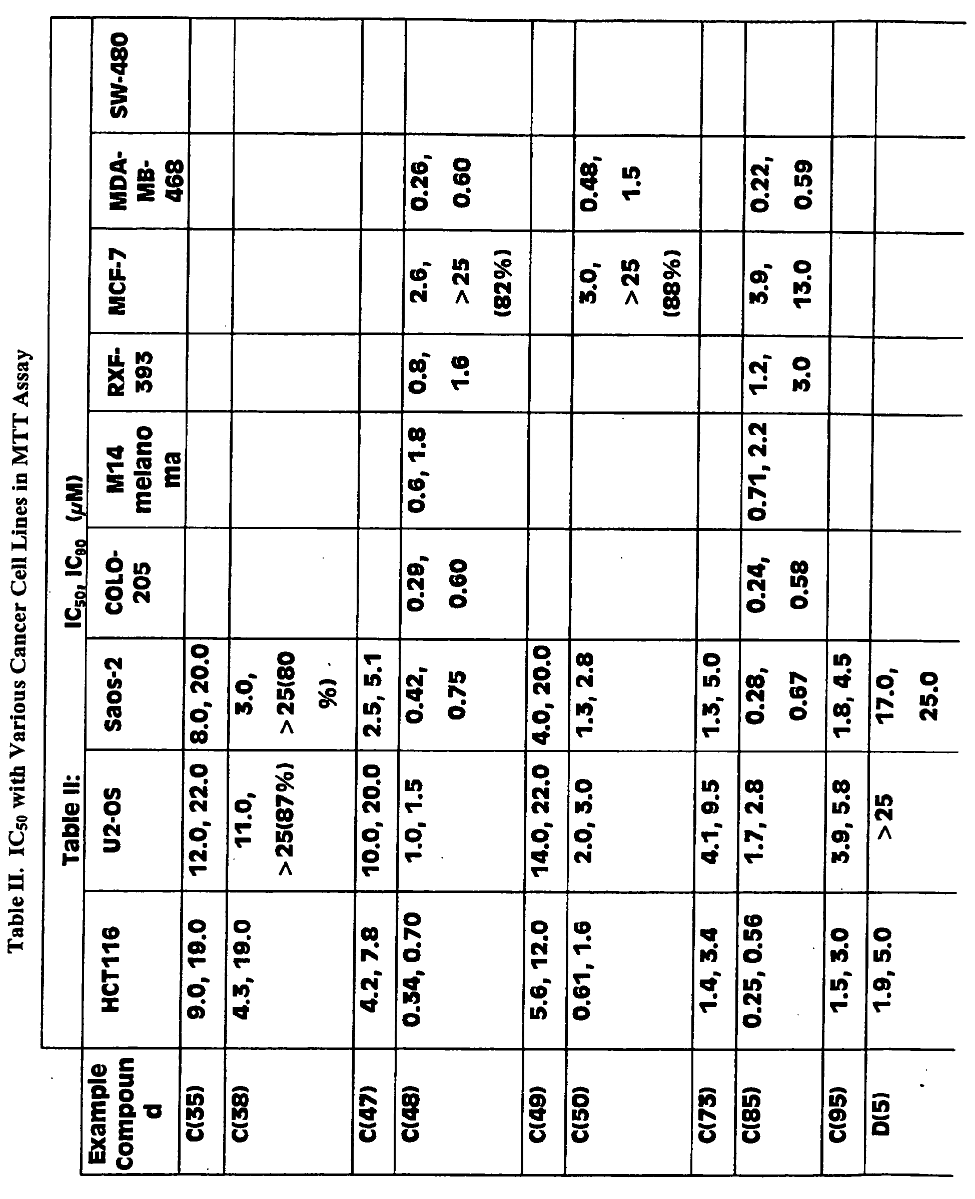

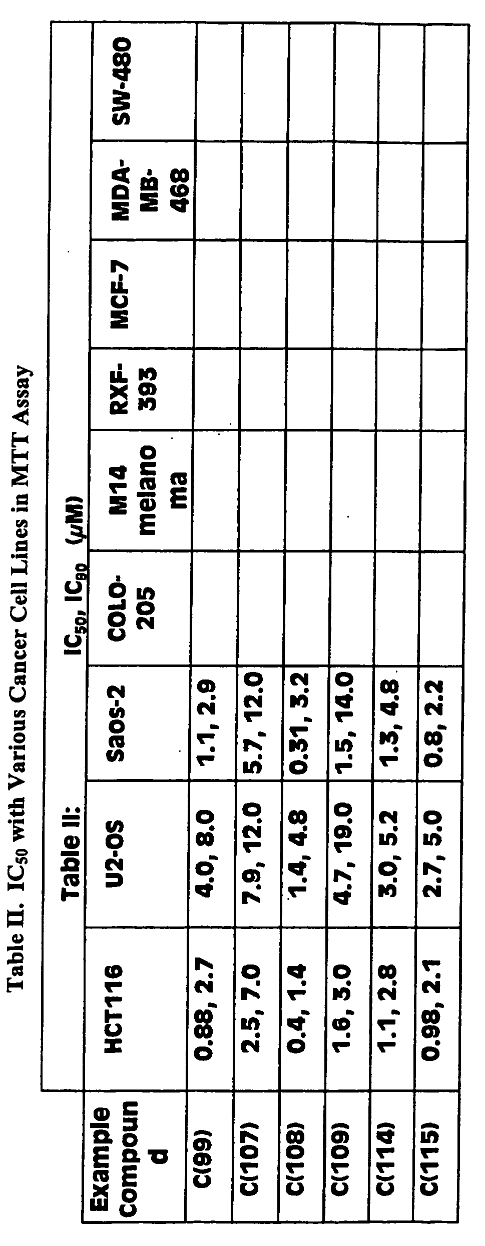

### pRb Immunoblotting:

The ability of compounds to inhibit phosphorylation of the retinoblastoma protein (pRb) was assessed by western blot analysis. An anti-Rb antibody was used to measure the conversion of hyper-phosphorylated pRb to hypo-phosphorylated pRb. An anti-phospho-Rb (ser780) antibody was used to specifically measure dephosphorylation at serine 780, a site that has previously been shown to be phosphorylated by CDK4/cyclin D. Inhibition of pRb phosphorylation is indicated by a "+" in Table III below, and failure to inhibit pRb phosphorylation is indicated by a "-" in the table.

Human colon tumor cells (HCT-116 cells; 5x10⁶) were plated on 100 mM dishes and allowed to grow overnight. Five micromolar of each compound was added for 12 hours. The cells were then collected and centrifuged. The cell pellets were lysed by the addition of 100 µL lysis buffer (50 mM HEPES (pH 7.0), 250 mM NaCl, 5 mM ethylenediaminetetraacetic acid, 0.1 % Nonidet P-40, 1 mM dithiothreitol, 2 mM sodium pyrophosphate, 1 mM sodium orthovanadate, 1 µg/ml aprotonin, 1 µg/ml leupeptin, 50 µg/ml phenylmethylsulfonyl fluoride). Forty micrograms of protein were separated by sodium dodecyl sulfate-polyacrylamide gel electrophoresis (SDS-PAGE) on a 6% gel. The proteins were transferred to nitrocellulose and blocked with 5% blocking buffer in Tris-buffered saline overnight. The anti-Rb antibody (Pharmingen), the anti-phospho-Rb (Ser 780) antibody (MBL), and secondary antibody were incubated for 1 hour at room temperature followed by three wash steps in 0.01 % Tween-20 in Tris-buffered saline. The Rb protein was detected using chemiluminescence according to the manufacturer (Amersham).

**Table III: Inhibition of pRb Phosphorylation**

| **Example Compound** | **Inhibits pRb phosphorylation** | **Inhibits pRb (ser 780) phosphorylation** |
|---|---|---|
| C(85) | + | + |
| C(48) | + | + |
| C(50) | + | + |
| C(73) | + | + |
| F | - | - |

The examples above illustrate compounds according to Formula I and assays that may readily be performed to determine their activity levels against the various CDK/cyclin complexes. It will be apparent that such assays or other suitable assays known in the art may be used to select an inhibitor having a desired level of activity against a selected target.

While the invention has been illustrated by reference to specific and preferred embodiments, those skilled in the art will recognize that variations and modifications may be made through routine experimentation and practice of the invention. For example, those of ordinary skill in the art will recognize that variations or substitutions to the compounds of Formula I may be made without adversely affecting in a significant manner their efficacy in the pharmaceutical compositions. Thus, the invention is intended not to be limited by the foregoing description, but to be defined by the appended claims and their equivalents.

## Claims

1. A compound of Formula I: 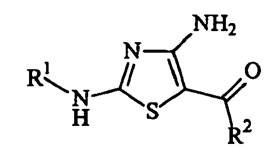 wherein R¹ is selected from: 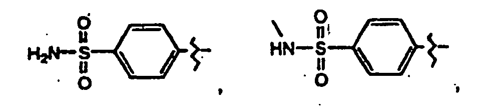 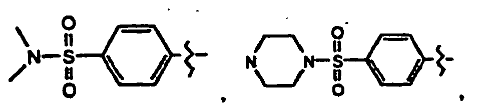 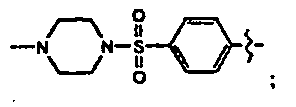 and R² is a substituted or unsubstituted carbocyclic or heterocyclic, monocyclic or fused or non-fused polycyclic ring structure, where each optional substituent for R² independently is a halogen, oxygen, haloalkyl, C₁₋₆-alkyl, C₁₋₆-alkenyl, C₁₋₆-alkinyl, hydroxyl, C₁₋₆-alkoxyl, carbocyclic or heterocyclic, monocyclic or fused or non-fused polycyclic cycloalkyl; carbocyclic or heterocyclic, monocyclic or fused or non-fused polycyclic aryl, amio, nitro, thiol, thioether, imine, cyano, amido, phosphonato, phosphine, carboxyl, thiocarbonyl, sulfonyl, sulfonamide, ketone, aldehyde or ester
or a pharmaceutically acceptable salt of a compound of Formula I.

2. A compound according to claim 1, selected from the group consisting of: 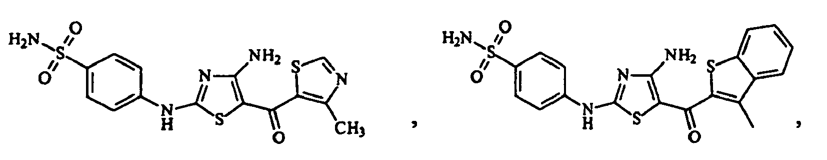 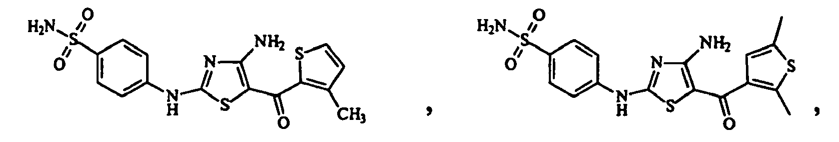 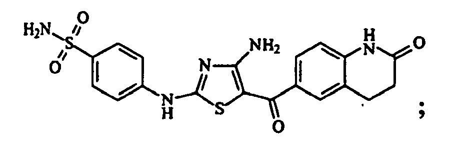 or a pharmaceutically acceptable salt of said compound.

3. A pharmaceutical composition comprising:
(a) an effective amount for inhibiting a CDK or a CDK/cyclin complex of a cell-cycle control agent, said cell-cycle control agent being selected from
(i) the compound of Formula I as defined in Claim 1; and
(ii) a pharmaceutically acceptable salt of a compound of the Formula I; and
(b) a pharmaceutically acceptable carrier.

## Patentansprüche

1. Verbindung der Formel I: 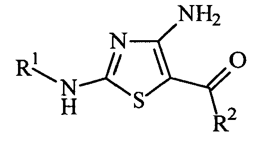 wobei R¹ ausgewählt ist aus: 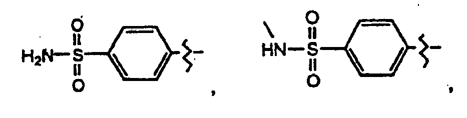 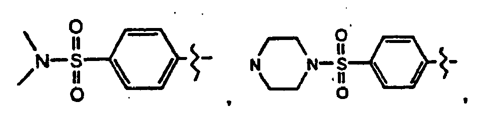 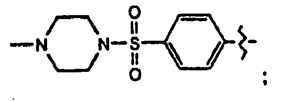 und R² eine substituierte oder unsubstituierte carbozyklische oder heterozyklische, monozyklische oder kondensierte oder nicht-kondensierte polyzyklische Ringstruktur ist, wobei jeder optionale Substituent für R² unabhängig voneinander ein Halogen, Sauerstoff, Haloalkyl, C₁₋₆-Alkyl, C₁₋₆-Alkenyl, C₁₋₆-Alkinyl, Hydroxyl, C₁₋₆₋Alkoxyl, carbozyklisches oder heterozyklisches, monozyklisches oder kondensiertes oder nicht-kondensiertes polyzyklisches Cycloalkyl; carbozyklisches oder heterozyklisches, monozyklisches oder kondensiertes oder nicht-kondensiertes polyzyklisches Aryl, Amio, Nitro, Thiol, Thioether, Imin, Cyano, Amido, Phosphonato, Phosphin, Carboxyl, Thiocarbonyl, Sulfonyl, Sulfonamid, Keton, Aldehyd oder Ester
oder ein pharmazeutisch akzeptables Salz einer Verbindung der Formel I ist.

2. Verbindung nach Anspruch 1, ausgewählt aus der Gruppe bestehend aus: 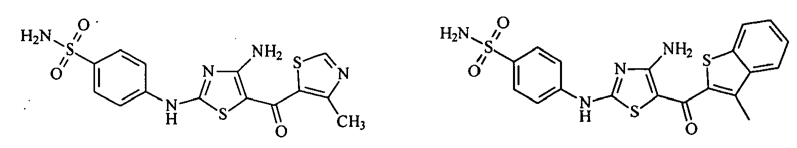 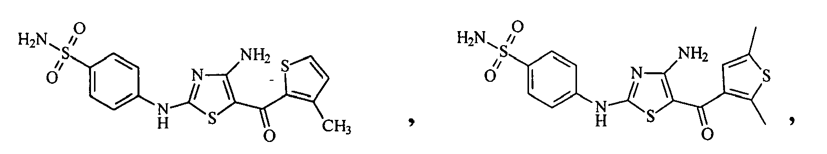 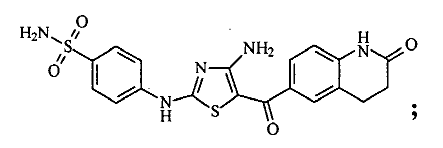 oder ein pharmazeutisch akzeptables Salz der Verbindung.

3. Pharmazeutische Zusammensetzung, umfassend:
(a) eine effective Menge zum Inhibieren eines CDK- oder eines CDK/Cyclin-Komplexes eines Zellzyklus-Steuerungs-Agens, wobei das Zellzyklus-Steuerungs-Agens ausgewählt ist aus
(i) der Verbindung nach Formel I, wie in Anspruch 1 definiert; und
(ii) einem pharmazeutisch akzeptablen Salz einer Verbindung der Formel I; und
(b) einem pharmazeutisch akzeptablen Träger.

## Revendications

1. Conmposé de formule I: 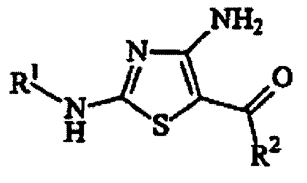 dans lequel R¹ est choisi parmi : 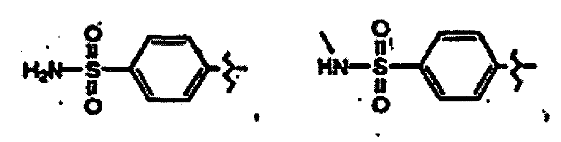 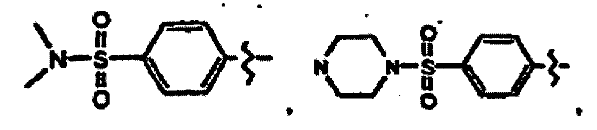 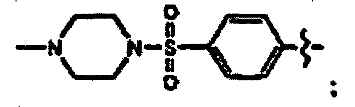 et R² est un noyau polycyclique condensé ou non condensé ou monocyclique, hétérocyclique ou carbocyclique substitué ou non substitué, où chaque substituant pour R² est indépendamment un cycloalkyle polycyclique condensé ou non condensé ou monocyclique, carbocyclique ou hétérocyclique, alcoxyle en C₁ à C₆, hydroxyle, alcynyle en C₁ à C₆, alcényle en C₁ à C₆, alkyle en C₁ à C₆, haloalkyle, oxygène, halogène; aryle polycyclique condensé ou non condensé ou monocyclique, carbocyclique ou hétérocyclique, amino, nitro, thiol, thioéther, imine, cyano ; amido, phosphonato, phosphine, carboxyle, thiocarbonyle, sulfonyle, sulfonamide, cétone, aldéhyde ou ester
ou un sel pharmaceutiquement acceptable d'un composé de formule I.

2. Composé selon la revendication 1, sélectionné dans le groupe constitué par : 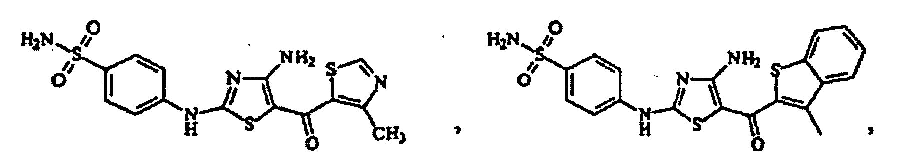 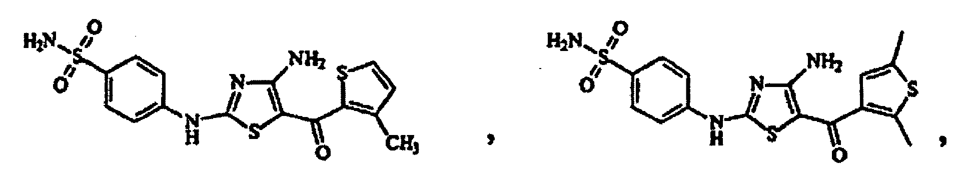 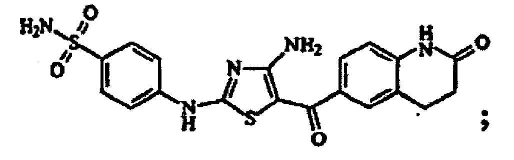 ou sel pharmaceutiquement acceptable dudit composé.

3. Composition pharmaceutique comprenant:
(a) une quantité efficace d'un agent de contrôle du cycle cellulaire pour inhiber un complexe cycline/CDK ou une CDK, ledit agent de contrôle du cycle cellulaire étant sélectionné à partir :
(i) de composé de formule I comme défini dans la revendication 1 ; et
(ii) d'un sel pharmaceutiquement acceptable d'un composé de la formule I ; et
(b) un véhicule pharmaceutiquement acceptable.
